# EUROPEAN PATENT SPECIFICATION

(11) **EP 3 509 554 B1**
(45) Date of publication and mention of the grant of the patent: **23.06.2021**
(21) Application number: 17777685.3
(22) Date of filing: 08.09.2017
(51) Int. Cl.: A61F 13/511

(54) **SYSTEMS AND METHODS OF APPLYING COMPOSITIONS TO WEBS AND WEBS THEREOF**
SYSTEME UND VERFAHREN ZUM AUFTRAGEN VON ZUSAMMENSETZUNGEN AUF BAHNEN UND BAHNEN DARAUS
SYSTÈMES ET PROCÉDÉS D'APPLICATION DE COMPOSITIONS SUR DES BANDES CONTINUES ET BANDES CONTINUES EN RÉSULTANT

(30) Priority: 09.09.2016 US 201662385265 P
(43) Date of publication of application: 17.07.2019
(73) Proprietor: The Procter & Gamble Company, Cincinnati, OH 45202 (US)
(72) Inventor: AVILES, Misael, Omar, Cincinnati Ohio 45202 (US); JAMES, Martin, Ian, Cincinnati Ohio 45202 (US); VARGA, Steven, Michael, Cincinnati Ohio 45202 (US); HAMMONS, John, Lee, Cincinnati Ohio 45202 (US); ROSATI, Rodrigo, 65824 Schwalbach am Taunus (DE)
(74) Representative: P&G Patent Germany
(86) International application number: PCT/US2017/050603
(87) International publication number: WO 2018/049108

(56) References cited:
- WO-A1-2009/062998
- WO-A1-2015/015417
- WO-A1-2017/136614
- US-A1- 2003 171 729
- US-A1- 2015 250 662
- US-B2- 9 237 973

## Description

### FIELD OF THE INVENTION

The present invention pertains to the application of compositions to webs and the resultant webs thereof.

### BACKGROUND OF THE INVENTION

Nonwovens, films, and laminates thereof are widely used in disposable absorbent article manufacturing. For example, many commercially available disposable absorbent articles utilize a nonwoven topsheet and some may use a nonwoven / film laminate backsheet. Many of these articles comprise printing on the nonwoven and/or film.

Typically, it is desired for operations like printing to occur at the normal operating speed of the manufacturing line. As such, registration marks are often utilized in conjunction with vision systems to trigger certain operations. Typically, printing may be offset to some extent in a machine direction and to some extent in a cross machine direction. In general, any offset would be passed along to the entirety of the print design such that the entire print design would be offset. So as long as the offset in either the machine direction or the cross machine direction was not too great, the print design would appear in tolerance with respect to the article.

However, where printing is desired to be based upon particular features of the article, there is increased complexity. For example, where the printing is desired to coincide with the features, to overlap features, or to be spaced from features, an offset between the printing and the desired location could impact functionality and/or falsely highlight features which are not desired. As a specific example, where printing is desired to coincide with apertures in a topsheet of an article, any offset in the machine direction and/or cross machine direction can cause the printing to be offset from the aperture.

Based on the foregoing, there is a need for a process which can effectively deposit compositions based upon particular features on the web or vice versa.

WO 2017/136614 A1 discloses a treated three-dimensional apertured bodyside liner material. The liner material comprises a plurality of raised areas, with a hydrophobic treatment agent disposed on the raised areas.

### SUMMARY OF THE INVENTION

The present invention provides an absorbent article according to claim 1.

### BRIEF DESCRIPTION OF THE DRAWINGS

Figure 1A is a schematic diagram showing a process in accordance with the present disclosure.
Figure 1B is a schematic diagram showing another process in accordance with the present disclosure.
Figure 1C is a plan view schematic representation of an exemplary secondary web constructed in accordance with the present disclosure.
Figure 2 is a plan view schematic representation of the exemplary secondary web of Figure 1C with the provision of composition associated with discontinuities.
Figure 3A is an elevation view schematic representation of a discontinuity in accordance with the present disclosure.
Figure 3B is an elevation view schematic representation of another discontinuity in accordance with the present disclosure.
Figure 4A is a schematic representation of an exemplary process for producing secondary webs comprising discontinuities in accordance with the present disclosure.
Figure 4B is a cross-sectional schematic representation of a disposable absorbent article comprising the secondary web of the process of Figure 4A.
Figures 5A-5H are schematic representations of other discontinuities which may be comprised by secondary webs in accordance with the present disclosure.
Figures 6A-6B are schematic representations of other discontinuities in accordance with the present disclosure.
Figures 7A-7D are cross-sectional schematic representations of webs comprising discontinuities in accordance with the present disclosure.
Figure 7E is a perspective view schematic representation of a web comprising discontinuities in accordance with the present disclosure.
Figure 7F is a top view of a 25 gsm polyethylene film web with discontinuities (film is stretched/flattened out to show high and low basis weight regions).
Figure 7G is a top view of a 60 gsm polypropylene nonwoven web with discontinuities (nonwoven is stretched/flattened out to show high and low basis weight regions).
Figure 7H is a cross-section view of the web shown in Figure 7G.
Figure 7I is side perspective view of another nonwoven web with discontinuities.
Figure 7J is a top perspective view of another nonwoven web with discontinuities.
Figure 7K is an isometric view schematic representation of an exemplary material web with corrugations in accordance with the present disclosure.
Figure 7L is a close up view of a corrugation of the material web of Figure 7K.
Figure 7M is a perspective view schematic representation of a web comprising discontinuities in accordance with the present disclosure.
Figure 8 is a cross-sectional schematic representation showing a web in accordance with the present disclosure fusion bonded to another web.

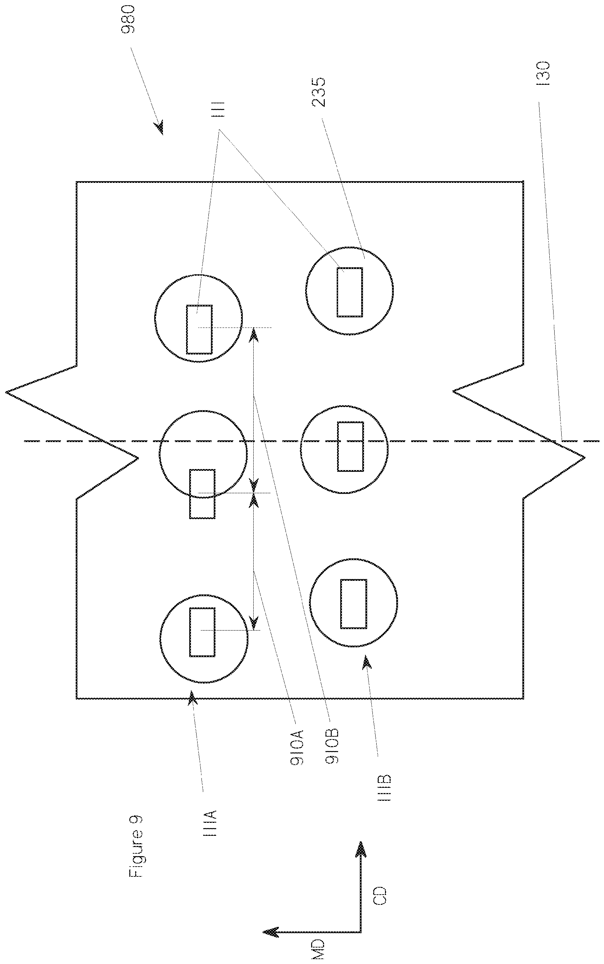

Figure 9 is a plan view schematic representation of a web in accordance with the present disclosure.
Figure 10 is a schematic diagram showing another process in accordance with the present disclosure.
Figure 11 is a schematic diagram showing another process in accordance with the present disclosure.
Figures 12A and 12B are schematic diagrams showing other processes in accordance with the present disclosure.
Figure 13 is a schematic diagram showing another process in accordance with the present disclosure.
Figure 14 is a plan view schematic representation of another web in accordance with the present disclosure.
Figure 15 is a schematic representation of a composition site in accordance with the present disclosure.
Figure 16 is a schematic representation of a composition site in accordance with the present disclosure.
Figure 17 is a cross-sectional schematic representation of a disposable absorbent article in accordance with the present disclosure.
Figures 18-20 are photographs showing a portion of a formed web comprising three-dimensional structures.
Figure 21 is a front view of a portion of a three-dimensional, liquid permeable substrate, wearer-facing surface facing the viewer in accordance with the present disclosure.
Figure 22 is a front perspective view of the portion of the three-dimensional, liquid permeable substrate of Figure 21 in accordance with the present disclosure.
Figure 23 is another front view of a portion of a three-dimensional, liquid permeable substrate, wearer-facing surface facing the viewer in accordance with the present disclosure.
Figure 24 is a front perspective view of the portion of the liquid permeable substrate of Figure 23 in accordance with the present disclosure.
Figure 25 is a back view of a portion of a three-dimensional, liquid permeable substrate, wearer-facing surface facing the viewer in accordance with the present disclosure.
Figure 26 is a back perspective view of the portion of the three-dimensional, liquid permeable substrate of Figure 25 in accordance with the present disclosure.
Figure 27 is another back view of a portion of a three-dimensional, liquid permeable substrate, wearer-facing surface facing the viewer in accordance with the present disclosure.
Figure 28 is a back perspective view of the portion of the liquid permeable substrate of Figure 27 in accordance with the present disclosure.
Figure 29 is a cross-sectional view of the liquid permeable substrate in accordance with the present disclosure.
Figures 30A-33B are photomicrographs depicting exemplary water droplets on fibers for the SEM contact angle measurement method disclosed herein.

### DETAILED DESCRIPTION OF THE INVENTION

The systems and methods of the present invention can facilitate the deposition of a composition or a plurality of compositions associated with discontinuities on a secondary web. For the purposes of the present disclosure, nonwoven webs, film webs, and laminates thereof will be generically referred to as a "web" unless otherwise expressed.

As used herein "hydrophilic" and "hydrophobic" have meanings as well established in the art with respect to the contact angle of a referenced liquid on the surface of a material. Thus, a material having a liquid contact angle of greater than about 90 degrees is considered hydrophobic, and a material having a liquid contact angle of less than about 90 degrees is considered hydrophilic. Compositions which are hydrophobic, will increase the contact angle of a referenced liquid on the surface of a material while compositions which are hydrophilic will decrease the contact angle of a referenced liquid on the surface of a material. Notwithstanding the foregoing, reference to relative hydrophobicity or hydrophilicity between a material and a composition, between two materials, and/or between two compositions, does not imply that the materials or compositions are hydrophobic or hydrophilic. For example, a composition may be more hydrophobic than a material. In such a case neither the composition nor the material may be hydrophobic; however, the contact angle exhibited by the composition is greater than that of the material. As another example, a composition may be more hydrophilic than a material. In such a case, neither the composition nor the material may be hydrophilic; however, the contact angle exhibited by the composition may be less than that exhibited by the material.

As used herein the term "print file" shall mean any streamed or batched electronic sequence provided to a printer such that all required rendering and formatting has been completed sufficient to allow the printer to execute a print pattern without further prerequisite processing or rendering. Various printers may require that the sequence be provided in specific formats. The sequences may have proprietary layers for either the protocols or the physical layers. Common examples include USB, USB 3.0, USB 3.1, Ethernet 10/100, Ethernet IP, GigE, CameraLink, Coax-Express, LVDS, TTL, RS485, RS422, and Serial Comm.; however, the printer may require its own unique protocols instead of industry common protocols.

The process pertains to the deposition of compositions onto a web. The composition deposition may include a plurality of composition sites which are based upon discontinuities. In some forms however, at least one composition site may be deposited on a web prior to the formation of the discontinuity. For example, in some forms of the present invention, one or more composition sites may be deposited on a web. Subsequently, the web may be manipulated thereby forming discontinuities.

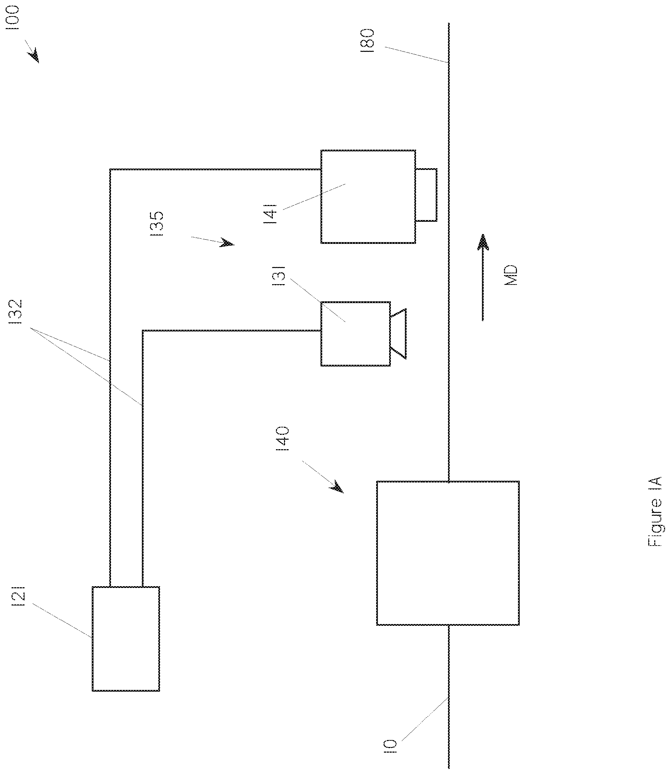

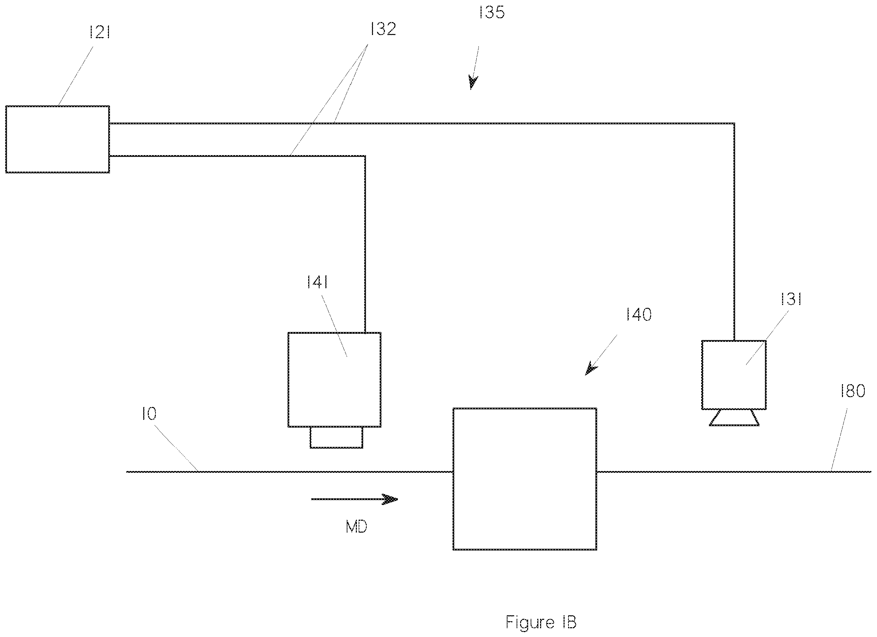

Figures 1A and 1B depict an exemplary apparatus 100 and process for carrying out methods of the present invention. The processes shown in Figures 1A and 1B allow for the deposition of one or more compositions relative to one or more discontinuities in a web of material. As shown, in some forms of the present invention, the apparatus 100 comprises a first unit operation 140 and an inspection / print station 135. As shown, a precursor web 10 may be provided to the first unit operation 140. As noted above, the precursor web 10 may comprise a nonwoven web, a film web, or a laminate created therefrom, e.g. nonwoven / nonwoven, film / film, nonwoven / film, or the like. Exemplary materials for precursor webs 10 are discussed hereafter.

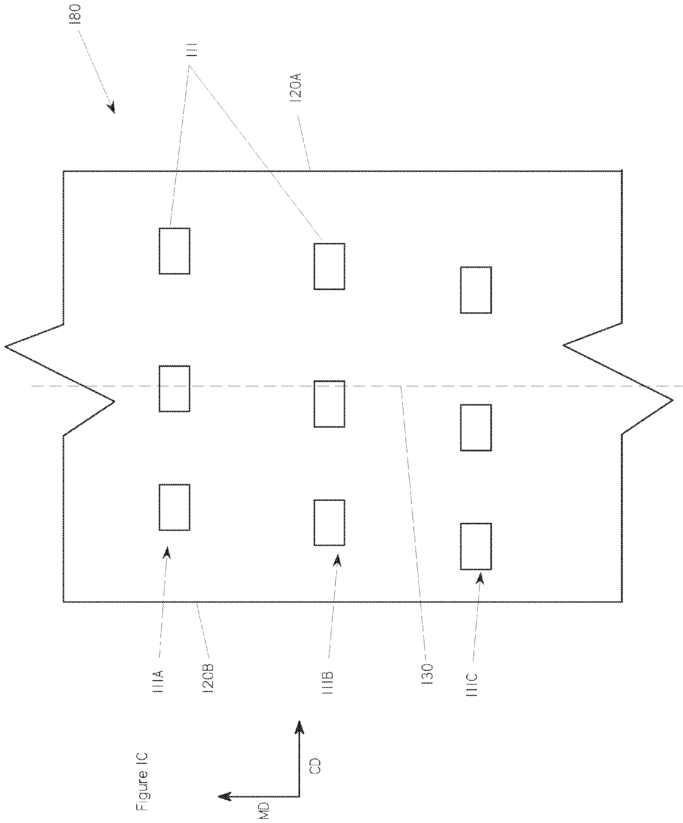

The first unit operation 140 may provide the precursor web 10 with a first plurality of discontinuities 111 (see Figure 1C) and may transform the precursor web 10 into a secondary web 180. With regard to Figure 1A, an inspection / print station 135 may inspect the secondary web 180 after the first plurality of discontinuities 111 have been provided to the precursor web 10.

While Figure 1A depicts a unit operation 140 which creates discontinuities, a web comprising discontinuities may be obtained from a supplier. In such instances, the need for the first unit operation 140 would be reduced if not eliminated. Forms of the present invention are contemplated where a web is obtained from a supplier comprising a first plurality of discontinuities while a manufacturer provided the web with a second plurality of discontinuities different from the first plurality. Still in other forms, the manufacturer may obtain a web from a supplier, the web comprising the first plurality and second plurality of discontinuities.

As shown, inspection / print station 135 may comprise a camera 131 which is in signal communication 132 with a computational device 121 and a printer 141 in signal communication with the computational device 121. The camera 131 may capture an image or multiple images of the secondary web 180 and transmit the image or images to the computational device 121. The computational device 121 analyzes the image or images and may provide a signal to the printer 141 such that composition may be deposited by the printer 141 onto the secondary web 140.

In some forms of the present invention, the composition provided by the printer 141 may be registered with at least a portion of the first plurality of discontinuities 111 (see Figure 1C). In some forms, the composition provided by the printer 141 may be offset with respect to the first plurality of discontinuities 111. In some forms, the composition provided by the printer 141 may overlap to at least some extent the discontinuities 111 of the secondary web. Additionally, in some forms, the printer 141 may deposit more than one composition onto the secondary web 180.

With regard to Figure 1B, in some forms, the inspection / print station 135, or a portion thereof, e.g. printer 141 may be provided upstream of the first unit operation 140. In such forms, the printer 141 may provide at least a first composition to the precursor web 10. The first unit operation 140 may be synchronized with the printer 141 such that the first plurality of discontinuities 111 created by the first unit operation 140 are associated with the composition applied to the precursor web 10. In such forms, the camera 131 may capture an image or images of the secondary web 180 and provide the image or images to the computational device 121. If the composition(s) are determined to be offset from their desired location, the computational device can adjust the signal sent to the printer 141. In conjunction therewith or independently thereof, the first unit operation 140 may be advanced or retarded such that the composition(s) are associated with the discontinuities 111 as desired. In such forms, particularly where the composition(s) applied by the printer 141 are highlighted on the secondary web 180, the images provided to the computational device 121 can provide information regarding location of the first plurality of discontinuities 111 and the composition(s) on the secondary web 180.

Forms of the present invention are contemplated where the camera 131 is positioned on the upstream side of the first unit operation 140. In such forms, the camera 131 may capture an image or images of the precursor web 10 and provide the image or images to the computational device 121. In such forms, the composition(s) provided by the printer 141 may be highlighted such that the location of the composition(s) may be determined. From their location, the first unit operation 140 may be advanced or retarded such that the composition(s) are associated with the discontinuities 111 in the secondary web 180 as desired.

Regarding Figures 1A and 1B, as shown, the printer 141 may be positioned upstream of the camera 131 or vice versa. For example, for the configuration of apparatus 100 in Figure 1A, the camera 141 may be upstream of the printer 141. In such forms, where the composition(s) deposited by the printer 141 are highlighted, the image(s) captured by the camera 131 can provide data regarding the location of the composition(s) with respect to the discontinuities 111. The computational device 121 may adjust the print signal provided to the printer 141 if the composition(s) are not in their desired location(s). As another example, for the configuration of apparatus 100 in Figure 1B, the camera 131 may be upstream of the printer 141. In such forms, the camera 131 may provide image(s) to the computational device 121 regarding the position of the precursor web 10, particularly with regard to CD tracking. The computational device 121 may adjust the print signal to the printer 141 as needed to ensure that the composition(s) provided to the precursor web 10 are in their desired location.

Referring to Figure 1C, after the first unit operation 140 (shown in Figures 1A and 1B), the secondary web 180 may comprise a least one discontinuity. For example, the secondary web 180 may comprise the first plurality of discontinuities 111 arranged in a plurality of groups, 111A, 111B, and 111C. As shown, the secondary web 180 may comprise side edges 120A and 120B each of which extend generally parallel to a machine direction ("MD"). A cross machine direction ("CD") extends generally perpendicular to the MD and in the same plane as the MD and the secondary web 115.

As noted previously, the web can track in the CD direction as the web moves through the apparatus 100 (shown in Figures 1A and 1B). Accordingly, the first plurality of discontinuities 111 may comprise a phase shift with respect to a machine centerline 130 as the precursor web 10 (shown in Figures 1A and 1B) tracks through apparatus 100. For example, the first plurality of discontinuities 111 comprised by a first group 111A may be positioned at a phase shift of zero degrees. This means that the first plurality of discontinuities 111 are positioned where they were intended to be with respect to the secondary web 180. However, due to web tracking in the CD, a second group 111B may comprise a phase shift of positive 30 degrees as these discontinuities 111 are shifted slightly to the left of the machine centerline 130. And, a third group 111C may comprise a phase shift of positive 45 degrees as these discontinuities 111 are shifted to the left of the machine centerline 130 to a greater extent than the second group 111B. The phase shift may comprise a negative value as well. For example, where a group of the first plurality of discontinuities 111 are shifted to the right of the machine centerline 130, this group of discontinuities 111 would comprise a negative phase shift, e.g. negative 15 degrees.

And, for those forms requiring the composition(s) to be highlighted for location determination, the composition sites may be evaluated in the same manner as described above. Namely, the phase shift of the composition sites may be evaluated.

It is worth noting that the machine centerline 130 is a fixed reference. The discontinuities described herein are not required to straddle the centerline. For example, the discontinuities may be - by design - spaced from the machine centerline 130. In such cases, the discontinuities would be evaluated regarding their predetermined location from the machine centerline 130. Any offset from the predetermined location would be evaluated as a phase shift of greater than or less than zero.

Referring again to Figures 1A and 1B, the computational device 121 analyzes the transmitted image or images provided by the camera 131 to detect the first plurality of discontinuities 111 of the submitted image(s) and determine the phase shift of the first group 111A, the second group 111B, and/or the third group 111C of the first plurality of discontinuities 111. The determination of phase shift is discussed hereafter. The camera 131 may capture an image of the first group 111A of the first plurality of discontinuities 111. As another example, the camera 131 can capture an image(s) of the first group 111A, the second group 111B, and/or third group 111C of the first plurality of discontinuities 111. In some forms, the camera 131 may capture an image of at least a portion of the first group 111A, second group 1101B and/or third group 111C of the first plurality of discontinuities 111. The camera 131 may transmit the image of the first group 111A, the second group 111B and/or the third group 111C, or at least a portion(s) thereof, to the computational device 121. The determination of phase shift of the first plurality of discontinuities 111 by the computational device 121 is discussed hereafter.

After the determination of the phase shift of the first group 111A, second group 111B and/or third group 111C, the computational device 121 may compare the determined phase shift to a plurality of stored pre-rendered patterns. The computational device 121 may then choose which of the stored patterns most closely correlates to the determined phase shift of the first group 111A, second group 111B, and/or third group 111C. The computational device 121 may then provide the chosen stored pattern to the printer 141 for the first group 111A, second group 111B, and/or third group 111C such that composition could be applied to the secondary web 180 in the case of Figure 1A or the precursor web 10 in the case of Figure 1B.

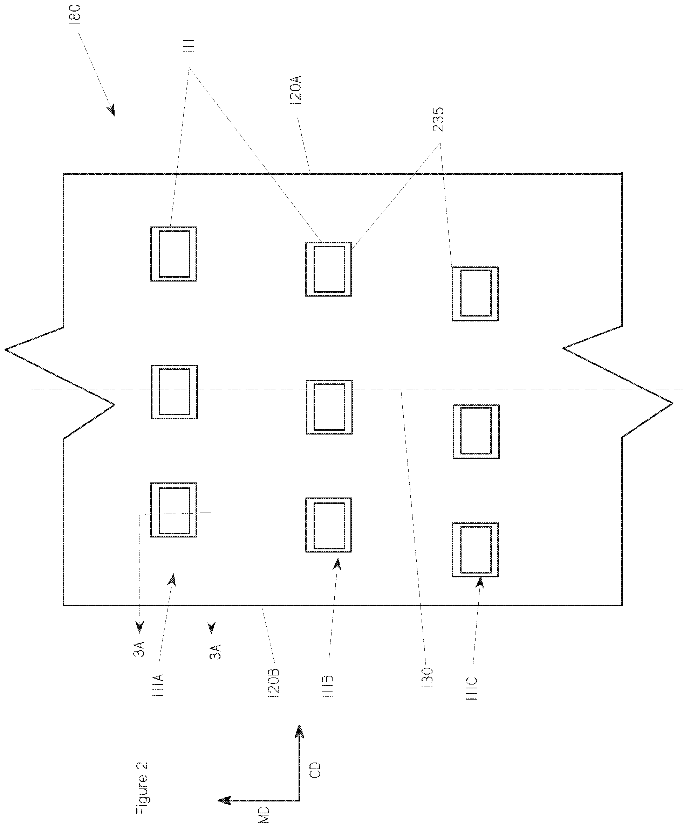

With the provision of the chosen stored pattern by the computational device 121, the printer 141 then applies composition to the precursor web 10 or the secondary web 180 depending on the orientation of the inspection / print station 135 disclosed herein. Regardless of the arrangement, the resultant secondary web 180 may comprise the first plurality of discontinuities 111 and a first plurality of composition sites 235 (shown in Figure 2). The determination of the phase shift of discontinuities on a web is discussed in additional detail in U.S. Patent Application Serial No. 62/291709.

In some forms of the present invention, the camera 131 may provide images directly to the printer 141. For example, as noted previously, the camera 131 may capture an image or image(s) with respect to the intermediate features and/or discontinuities. The camera 131 may then provide the image(s) directly to the printer 141 as a print file. The printer 141 may then apply compositions to the web in accordance with the image(s) provided by the camera 131. In such forms, there may be no need to have stored pre-rendered patterns for comparison.

As shown in Figure 2, the each of the first plurality of composition sites 235 is registered with corresponding discontinuities 111 of the first plurality of discontinuities 111. In some forms, composition may be applied to only a portion of the first plurality of discontinuities 111. In some forms, the first plurality of composition sites 235 may be disposed between adjacent discontinuities 111 in the first group 111A, second group 111B, and/or third group 111C. In some forms, the first plurality of composition sites 135 may be disposed between adjacent groups of discontinuities 111, e.g. between the first group 111A and second group 111B and/or between the second group 111B and third group 111C.

In some forms, the first plurality of composition sites 135 may be based upon the determined phase shift of the first group 111A of discontinuities 111. The printer 141 may also deposit a second plurality of composition sites according to a second stored pre-rendered pattern. The second plurality of composition sites may be based upon the determined phase shift of the second group 111B of discontinuities 111. In some forms, the first stored pre-rendered pattern may be different than the second stored pre-rendered pattern. Additionally, the printer 141 may also deposit a third plurality of composition sites according to a third stored pre-rendered pattern. The third plurality of composition sites may be based upon the determined phase shift of the third group 111C of discontinuities 111. In some forms, the first stored, pre-rendered pattern, the second stored, pre-rendered pattern, and/or the third stored, pre-rendered pattern may be different.

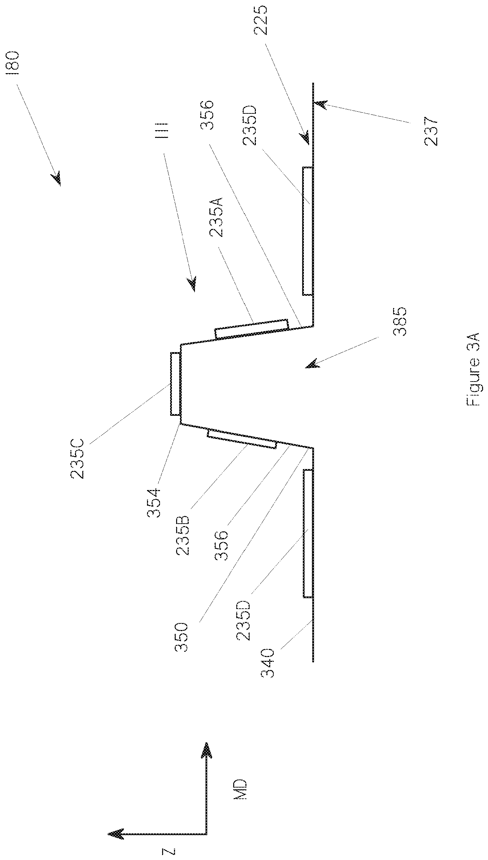

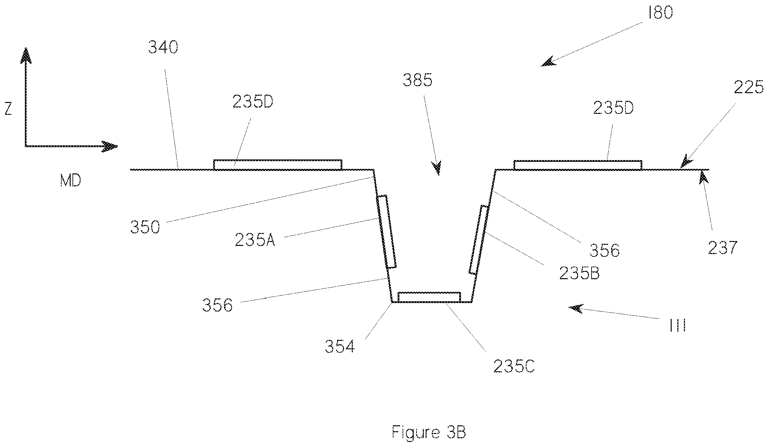

Referring to Figures 3A and 3B, while the discontinuities may take on many of several forms, which are discussed hereafter, for those discontinuities which extend from a first surface 225 or a second surface 237 of the secondary web 180, each have a similar macro structure. For example, some of the discontinuities 111 of the present disclosure protrude in either a positive Z-direction (Figure 3A) or in a negative Z-direction (Figure 3B). As shown in Figure 3A, the discontinuity 111 extends from the first surface 225 of the secondary web 180 in the positive Z-direction. In Figure 3B, the discontinuity 111 extends from the second surface 237 in the negative Z-direction. Regardless of orientation, each of the discontinuities 111 comprise a distal end 354 and sidewalls 356 which extend between the first surface 225 and the distal end 354 (see Figure 3A) or between the second surface 237 and the distal end 354 (see Figure 3B). Each of the discontinuities 111 comprises a base 350 which is where the sidewalls 356 are connected to the secondary web 180. Additionally, for each of the discontinuities 111, an opening or depression 385 corresponds with each discontinuity 111.

Each of the discontinuities 111 of the present disclosure may comprise at least one composition site. For example, a composition site 235C may be provided on the distal ends 354 of the discontinuities 111. As another example, composition site 235A and/or 235B may be provided on the sidewalls 356 of the discontinuity. As yet another example, composition sites 235D may be provided on a land area 340 between adjacent discontinuities 111. Forms of the present invention are contemplated where secondary webs 180 of the present invention comprise at least one of the composition sites 235A, 235B, 235C, 235D, or any combination thereof. Additionally, forms of the present invention are contemplated where each of the composition sites 235A, 235B, 235C, 235D comprise a different compositions or wherein at least two or at least three of the foregoing composition sites comprise differing compositions.

As shown in Figure 3A, the composition sites 235A, 235B, 235C, 235D may be applied to the first surface 225 of the precursor or secondary web 180. Note that as shown in Figure 3A, the first surface 225 may form an outer-facing surface of the discontinuity 111 and the composition sites 235A, 235B, 235C and 235D may be disposed on the first surface 225 / outer-facing surface of the discontinuity 111. Additional forms of the present invention are contemplated where a second printer is utilized in the apparatus 100 (shown in Figures 1A and 1B) and where the second printer engages the second surface 237 of the secondary web 180 or the second surface of the precursor web. In such forms, composition(s) may be provided to the second surface 237 and possibly on inner-facing surfaces of the discontinuity 111 along with the outer-facing surface as discussed above. Forms of the present invention are contemplated where composition sites are provided on the inner-facing surfaces of the discontinuities 111 sans the application of composition sites on the outer-facing surface of the discontinuities 111 or vice versa.

As shown in Figure 3B, the composition may be applied to the first surface 225 of the precursor or secondary web 180 as noted above. However, because the discontinuity 111 is protruding in the negative Z-direction, the first surface 225 may represent the inner-facing surfaces of the discontinuity, e.g. inner-facing surface 111A and 111B of sidewalls 356 and inner-facing surface 111C of the distal end 354. Similar to the above, a second printer may engage the second surface 237 of the precursor or secondary web 180. In such forms, composition(s) may be applied to the second surface / outer-facing surface of the discontinuity 111. The compositions sites may be applied to the outer-facing surface sans the application of composition sites on the inner-facing surface of the discontinuities 111 or vice versa.

It is worth noting that where the application of composition is desired on an inner-facing surface of the discontinuity 111, some additional challenges may present themselves. For example, as web processing is generally a continuous operation, it may be difficult to apply composition on the inner-facing surface of a discontinuity on a moving web. And, stopping the web, even for a brief instance, introduces additional cost to the manufacturing of the web. However, the arrangement of the printer in Figure 1B may overcome this problem in that composition(s) are provided to the precursor web prior to the formation of the discontinuities 111. Depending on the desired orientation of the discontinuities, a printer may apply composition to the first surface and/or the second surface of the precursor web at sites which will correspond with the sites 235A, 235B and/or 235C and the inner-facing surface of the discontinuity 111.

Referring back to Figure 3A, in some forms, the composition site 235C may comprise a hydrophobic composition. The composition sites 235A and 235B may comprise a hydrophobic composition as well while the composition site 235D comprises a hydrophilic composition. In some forms, the composition of composition site 235C may be more hydrophobic than the composition of the composition site 235A and/or 235B which may be more hydrophobic than the composition of composition site 235D.

Referring back to Figure 3B, in some forms, the composition site 235C may comprise a hydrophilic composition. The composition sites 235A and 235B may comprise a hydrophilic composition while the composition site 235D comprises a hydrophobic composition. In some forms, the composition of composition site 235C may be more hydrophilic than the composition of composition sites 235A and 235B and may be more hydrophilic than the composition of composition site 235D.

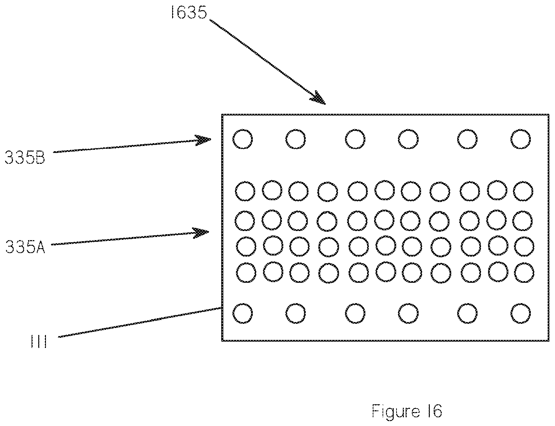

Specific forms of the present invention are provided with regard to prophetic examples E13 through E23. For each of the prophetic examples E13 though E23, the discontinuities of the present disclosure are oriented in the negative Z-direction, as shown in Figure 3B. Additionally, for these prophetic examples, the discontinuities, particularly their respective distal ends, may be oriented toward a garment-facing surface on an absorbent article. Additional details of prophetic examples E13 through E23 are provided below with reference to Figure 3B. It is important to recall that the composition sites 235A, 235B, 235C are shown as being on the inner surface of the discontinuity 111 unless otherwise mentioned.
E13: In such forms, a plurality of composition sites 235C may comprise a hydrophilic composition, e.g. a surfactant.
E14: In such forms, a plurality of composition sites 235C may comprise a blood modifying agent as disclosed herein.
E15: In such forms, a plurality of the composition sites 235A, 235B, and 235C may comprise a hydrophilic composition. The hydrophilic compositions applied to sites 235A, 235B, and/or 235C may be different from one another.
   (A) If applied via ink jet printing, the hydrophilic composition may be applied as a plurality of composition droplets or dots. The droplets may be configured such that the dots per inch, "DPI" of the composition site 235C are the same or greater than the DPI of the composition sites 235A or 235B.
   (B) The hydrophilic composition may be applied to a plurality of distal ends 354 via a contact method, e.g. slot coating, as described herein. In such forms, the compositions of the composition sites 235A or 235B may similarly deposited via contact methods or via ink jet printing.
E16: In such forms, a plurality of the composition sites 235C may comprise a blood modifying agent and a plurality of the composition sites 235A and 235B may comprise a hydrophilic composition, e.g. a surfactant.
E17: In such forms, a plurality of composition sites 235D may comprise a hydrophobic composition.
   (A) If applied via ink jet printing, each of the plurality of composition sites 235D may comprise a first portion and a second portion (as discussed with regard to Figure 16) where the first portion is disposed adjacent openings 385 of a plurality of discontinuities, and the second portion is outboard of the first portion. In such forms, the DPI of the first portion may be the same or greater than that of the second portion.
   (B) If applied via ink jet printing, each of the plurality of composition sites 235D may comprise a first portion and a second portion (as discussed with regard to Figure 16) where the first portion is disposed adjacent openings 385 of a plurality of discontinuities, and the second portion is outboard of the first portion. In such forms, the DPI of the second portion may be the same or greater than that of the first portion.
E18: In such forms, a plurality of composition sites 235A, 235B, 235C may comprise a blood modifying agent or a hydrophilic composition while a plurality of composition sites 235D comprise a hydrophobic composition.
   (A) If applied via ink jet printing, the blood modifying agent may be applied as a plurality of composition droplets or dots. The droplets may be configured such that the dots per inch, "DPI" of the composition site 235C are the same or greater than the DPI of the composition sites 235A or 235B.
   (B) If applied via ink jet printing, each of the plurality of composition sites 235D may comprise a first portion and a second portion (as discussed with regard to Figure 16) where the first portion is disposed adjacent openings 385 of a plurality of discontinuities, and the second portion is outboard of the first portion. In such forms, the DPI of the first portion may be the same or greater than that of the second portion. This form is contemplated independent of (A) or (D) or may be done in conjunction with (A) or (D) of E18.
   (C) If applied via ink jet printing, each of the plurality of composition sites 235D may comprise a first portion and a second portion (as discussed with regard to Figure 16) where the first portion is disposed adjacent openings 385 of a plurality of discontinuities, and the second portion is outboard of the first portion. In such forms, the DPI of the second portion may be the same or greater than that of the first portion. This form is contemplated independent of (A) or (D) or may be done in conjunction with (A) or (D) of E18.
   (D) The blood modifying agent may be applied to a plurality of distal ends 354 via a contact method, e.g. slot coating, as described herein. In such forms, the compositions of the composition sites 235A or 235B may similarly deposited via contact methods or via ink jet printing.
E19: In such forms, a plurality of the composition sites 235C may comprise a hydrophilic composition or a blood modifying agent, and a plurality of the composition sites 235D may comprise a hydrophobic composition.
   (A) If applied via ink jet printing, each of the plurality of composition sites 235D may comprise a first portion and a second portion (as discussed with regard to Figure 16) where the first portion is disposed adjacent opening 385 of a plurality of discontinuities, and the second portion is outboard of the first portion. In such forms, the DPI of the first portion may be the same or greater than that of the second portion.
   (B) If applied via ink jet printing, each of the plurality of composition sites 235D may comprise a first portion and a second portion (as discussed with regard to Figure 16) where the first portion is disposed adjacent openings 385 of a plurality of discontinuities, and the second portion is outboard of the first portion. In such forms, the DPI of the second portion may be the same or greater than that of the first portion.

For the prophetic examples of E20 through E21, the precursor web may be hydrophobic. For example, in some forms, the precursor web may comprise a hydrophobic melt additive. Webs comprising melt additives are discussed in additional detail in U.S. Patent Application Serial Nos. 14/849630 and 62/305726.
E20: In such forms, a plurality of the composition sites 235C may comprise a hydrophilic composition, e.g. a surfactant, or a blood modifying agent.
E21: In such forms, a plurality of the composition sites 235A, 235B, and 235C may comprise a hydrophilic composition or a blood modifying agent as disclosed herein.
   (A) If applied via ink jet printing, the hydrophilic composition may be applied as a plurality of composition droplets or dots. The droplets may be configured such that the dots per inch, "DPI" of the composition site 235C are the same or greater than the DPI of the composition sites 235A or 235B.
   (B) The hydrophilic composition may be applied to a plurality of distal ends 354 via a contact method, e.g. slot coating, as described herein. In such forms, the compositions of the composition sites 235A or 235B may similarly deposited via contact methods or via ink jet printing.

In contrast to the above prophetic examples E20 and E21, forms of the present invention are contemplated where the precursor web comprises a hydrophilic melt additive or is otherwise hydrophilic. Prophetic examples E22 through E23 are described based upon this condition of the precursor web.
E22: In such forms, a plurality of the composition sites 235D may comprise a hydrophobic composition, e.g. a lotion.
   (A) If applied via ink jet printing, each of the plurality of composition sites 235D may comprise a first portion and a second portion (as discussed with regard to Figure 16) where the first portion is disposed adjacent opening 385 of a plurality of discontinuities, and the second portion is outboard of the first portion. In such forms, the DPI of the first portion may be the same or greater than that of the second portion.
   (B) If applied via ink jet printing, each of the plurality of composition sites 235D may comprise a first portion and a second portion (as discussed with regard to Figure 16) where the first portion is disposed adjacent openings 385 of a plurality of discontinuities, and the second portion is outboard of the first portion. In such forms, the DPI of the second portion may be the same or greater than that of the first portion.
E23: In such forms, a plurality of the composition sites 235A, 235B, and/or 235C may comprise a blood modifying agent as disclosed herein.
   (A) If applied via ink jet printing, the blood modifying agent may be applied as a plurality of composition droplets or dots. The droplets may be configured such that the dots per inch, "DPI" of the composition site 235C are the same or greater than the DPI of the composition sites 235A or 235B.
   (B) If applied via ink jet printing, each of the plurality of composition sites 235D may comprise a first portion and a second portion (as discussed with regard to Figure 16) where the first portion is disposed adjacent openings 385 of a plurality of discontinuities, and the second portion is outboard of the first portion. In such forms, the DPI of the first portion may be the same or greater than that of the second portion. This form is contemplated independent of (A) or (D) or may be done in conjunction with (A) or (D) of E23.
   (C) If applied via ink jet printing, each of the plurality of composition sites 235D may comprise a first portion and a second portion (as discussed with regard to Figure 16) where the first portion is disposed adjacent openings 385 of a plurality of discontinuities, and the second portion is outboard of the first portion. In such forms, the DPI of the second portion may be the same or greater than that of the first portion. This form is contemplated independent of (A) or (D) or may be done in conjunction with (A) or (D) of E23.
   (D) The blood modifying agent may be applied to a plurality of distal ends 354 via a contact method, e.g. slot coating, as described herein. In such forms, the compositions of the composition sites 235A or 235B may similarly deposited via contact methods or via ink jet printing.

As noted previously, the discontinuities of the present invention generally comprise a similar macro structure but may have vastly different micro structure. Also, the discontinuities of the present invention may comprise a variety of different forms. Exemplary discontinuities for use with the present invention are provided herein.

### Embossments

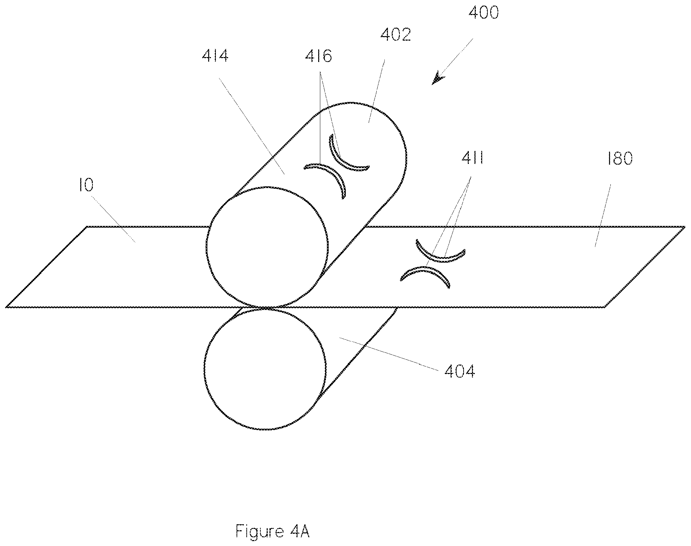

One specific example of a discontinuity for use in with the present invention comprises embossments. The first unit operation 140 (shown in Figures 1A and 1B) may comprise a process for forming embossments in the precursor web 10. Referring to Figure 4A, an apparatus 400 may comprise a forming roll 402 comprising a plurality of forming elements 416 and an anvil roll 404. The forming elements 416 of the forming roll 402 may protrude outward from a surface 414 of the forming roll 402. The anvil roll 404 may comprise a smooth outer surface. As the precursor web 10 travels between the forming roll 402 and anvil roll 404, embossments may be provided to the precursor web 10 yielding the secondary web 180.

In contrast to fusion bond sites, discussed hereafter, embossments 420 do not comprise the fusion of the constituent material of the secondary web 180 to adjacent materials. Instead, embossments 411 tend to compress the precursor web 10. Embossments 411 can vary acquisition rates in an absorbent article. For example, where the secondary web 180 forms a portion of a topsheet of an absorbent article, the embossment 411 may not readily receive a liquid insult. Instead, the embossment 411 may act as a liquid highway which can distribute the insult to multiple areas of an absorbent core in the absorbent article.

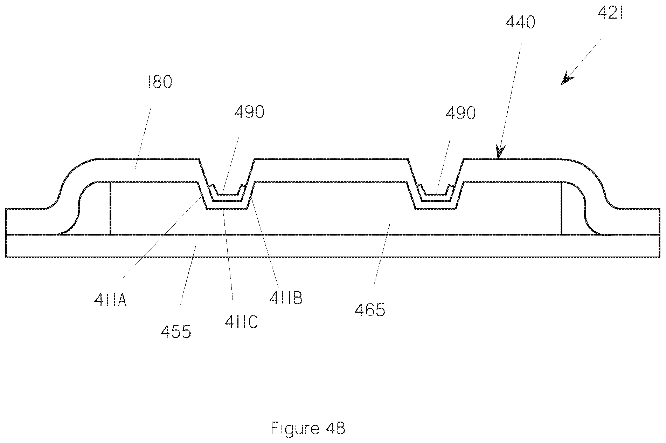

An exemplary cross section of the secondary web 180 in an absorbent article 421 is shown in Figure 4B. As shown, the absorbent article 421 comprises the secondary web 180 as a topsheet, a backsheet 455 and an absorbent core 465 disposed between the backsheet 455 and the topsheet (secondary web 180). In some forms, the secondary web 180 may comprise embossments 411, and adjacent the embossments 411 are land areas 440. In some forms, the secondary web 180 and the absorbent core 465 may comprise embossments 411. In some forms, the secondary web 180 and additional layers between topsheet and the absorbent core 465, e.g. acquisition layers, may comprise embossments 411.

Composition sites may be applied to the secondary web 180 as described heretofore with regard to Figures 3A and 3B. For example, referring back to Figure 4B, as shown, a plurality of composition sites 490 may be provided at distal ends 411C of the embossments. Additionally, the plurality of composition sites 490 may comprise portions which are disposed on sidewalls 411A and 411B of the embossment. In some forms, the plurality of composition sites 490 may comprise a hydrophobic composition. The compression which creates the embossments 411 can inhibit fluid acquisition in the embossment 411. A hydrophobic composition in the distal end 411C of the embossment 411 can assist in transporting liquid insults to additional areas of the absorbent article. Additionally, the hydrophobic composition can provide a cleaner look to the absorbent article in the area of the embossment 411 since the hydrophobic composition would discourage liquid insults from residing in the embossment 411.

Other forms of the present invention are contemplated where the composition sites 490 comprises a hydrophilic composition. In such forms, the hydrophilic composition may facilitate fluid acquisition by the embossments 411. It is worth noting however, that in such forms, the level of compression in the embossments 411 can offset the hydrophilic composition. For example, where the embossments 411 are formed with high compression, the embossments 411 have an increased density which generally inhibits fluid acquisition. In contrast, embossments 411 derived from lighter compression can drive better interaction between layers of the absorbent article 421 which can improve liquid acquisition. Forms of the present invention are also contemplated where composition sites are provided on the land areas 440 of the absorbent article 421. In such forms, these composition sites may have a similar composition or a different composition than what is provided in the composition sites 490.

Any suitable embossment pattern may be utilized in conjunction with the secondary web 180 of the present invention. Some suitable examples of embossment patterns are provided with regard to US Patent Nos. 6,170,393; 6,652,500; 7,056,404; 8,030,535; 8,492,609; 8,496,775; and U.S. Patent Application Publication Nos. 2013/0281953; and 2014/0031779.

### Tunnel Tufts / Filled Tufts

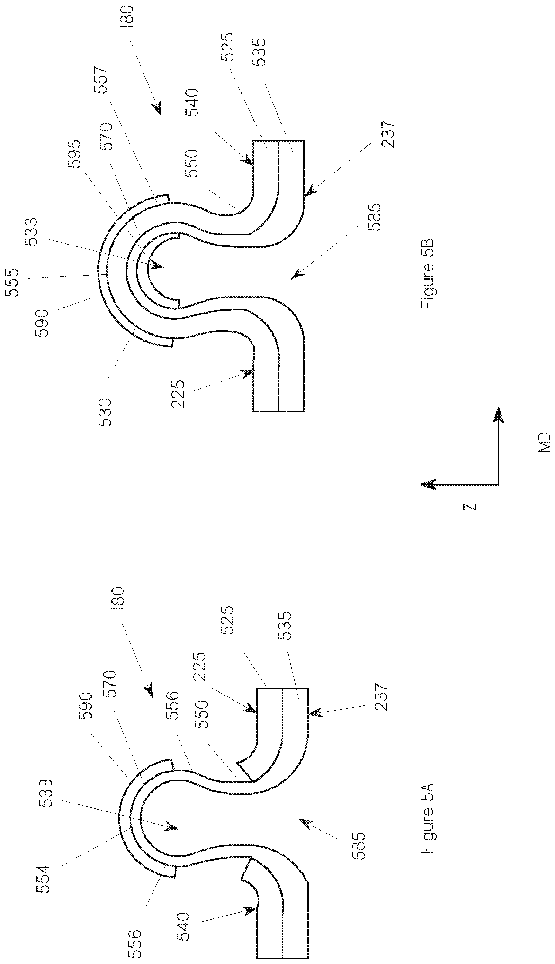

The discontinuities of the present invention may comprise tunnel tufts or filled tufts as provided herein. The tunnel tufts or filled tufts may be utilized in conjunction with the embossments described herein. Referring to Figures 5A-5H, in another specific example, the first unit operation 140 (shown in Figures 1A and 1B) may comprise an apparatus for forming tufts in the precursor web 10. The apparatus and method for forming the tunnel tufts / filled tufts are described in U.S. Patent Nos. 7,172,801; 7,838,099; 7,754,050; 7,682,686; 7,410,683; 7,507,459; 7,553,532; 7,718,243; 7,648,752; 7,732,657; 7,789,994; 8,728,049; 8,153,226; and in U.S. Patent Application Publication Nos. 2016/0067118 and 2016/0167334.

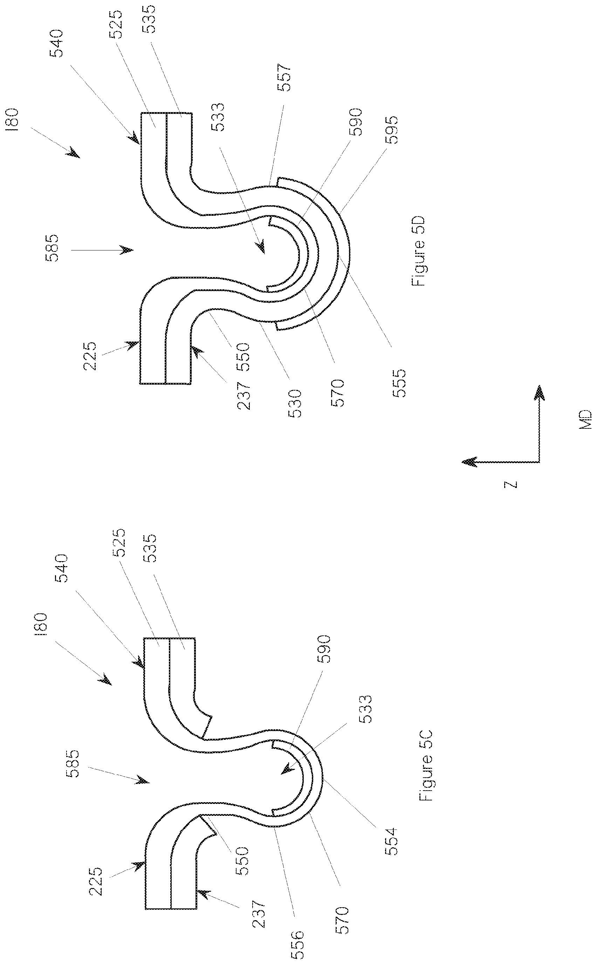

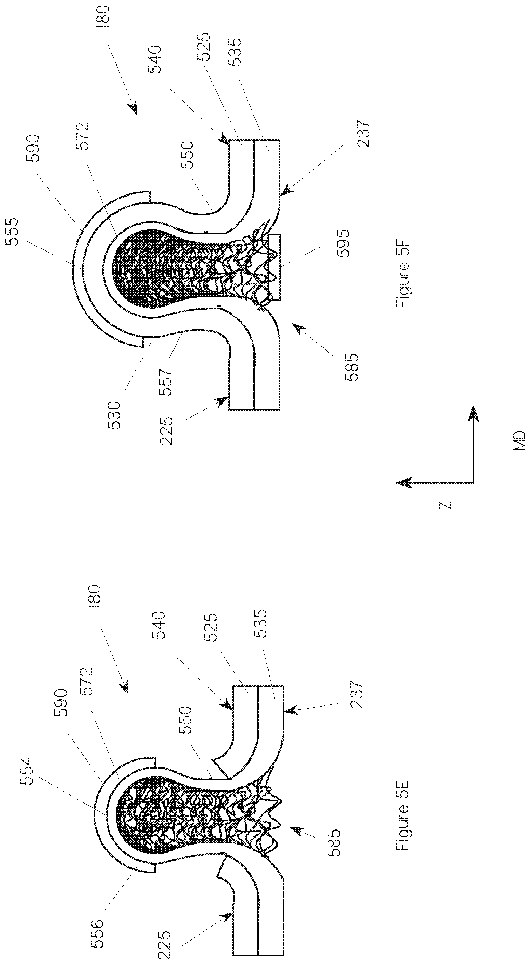

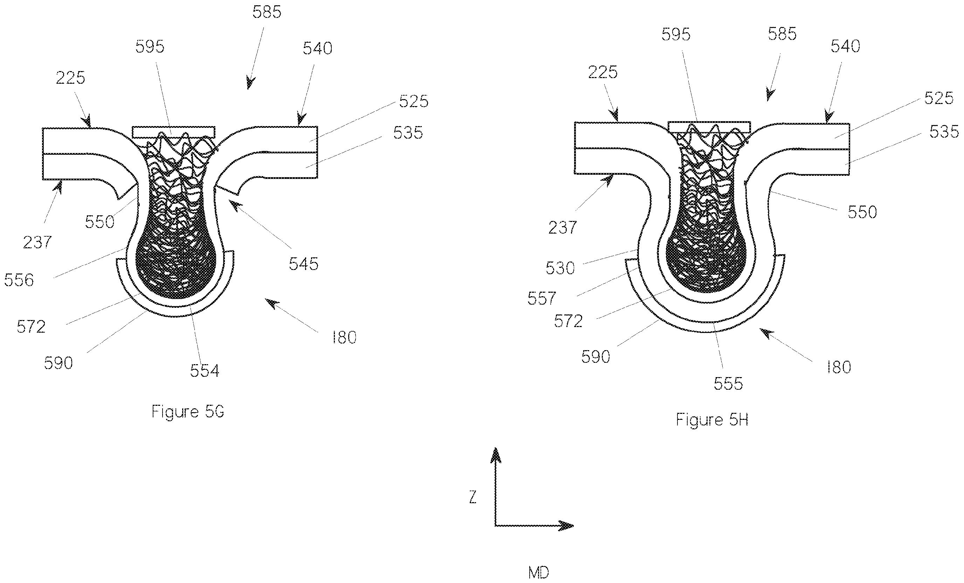

The apparatus described in the publications above can urge the material of the precursor web in the positive Z-direction (see Figures 5A, 5B, 5E, and 5F) or negative Z-direction (see Figures 5C, 5D, 5G, and 5H). The resultant structure of the secondary web 180 can comprise tunnel tufts, described with regard to Figures 5A-5D or may comprise filled tufts as described with regard to Figures 5E-5H. For the sake of clarity, the secondary web 180 depicted in Figures 5A-5H comprises multiple layers, e.g. first layer 525 and second layer 535, or multiple strata; however, forms of the present invention are contemplated where the secondary web 180 comprises only a single layer or a single strata.

As shown, each of the tunnel tufts 570, filled tufts 572, and outer tufts 530 comprise a base 550, a distal end 554, 555 spaced from the base 550, and sidewalls 556, 557 between the base 550 and the distal end 554, 555. As shown, a composition site 590 may be associated with the tunnel tuft 570, filled tuft 572, or with the outer tuft 530. An opening 585 in the second layer 535 or strata or in the first layer 525 or strata generally corresponds to the outer tuft 530, tunnel tuft 570, or filled tuft 572.

In the forms shown in Figures 5A, 5B, 5E, and 5F where the tunnel tufts 570, filled tufts 572, and outer tufts 530 are oriented in the positive Z-direction, the composition site 590 may be provided on the distal end 554, 555 of the tufts 570, 572, 530, respectively, and on a portion of the sidewalls 556 and 557 of the tuft 570, 572, and 530, respectively. Where the secondary web 180 is utilized as a topsheet, the composition site 590 may comprise a hydrophobic composition. In such forms, the composition can provide a reduction in rewet while providing adequate liquid acquisition. Additionally, in such forms, the composition site 590 may help with masking of liquid insults to a disposable absorbent article.

Referring specifically to Figure 5B, in some forms, composition sites, e.g. 595 and 590, may be provided to the secondary web 180. For example, as shown, composition site 590 may be provided to the outer tuft 530 while composition site 595 is provided to the tuft 570. In such forms, the composition site 595 may comprise a hydrophilic composition and the composition site 590 may comprise a hydrophobic composition. In some forms, the composition site 590 and the composition site 595 may each comprise hydrophobic compositions. As shown, the composition site 590 may be disposed on the distal end 555 of the outer tuft 530 and a portion of side walls 557 of the outer tuft 530. In some forms, the composition site 595 may be disposed on an inner surface of the tuft 570. Similarly, in such forms, where the secondary web 180 is utilized as a topsheet of an absorbent article, the above configuration can allow for sufficient liquid acquisition time while reducing rewet. Such configurations may additionally provide a benefit in masking liquid insults. Forms of the present invention are contemplated where composition sites are provided in land areas 540 of the secondary web 180. In such forms, the composition in the land areas 540 may be the same or different than the compositions in the composition sites 590 and/or 595. Composition sites 595 may similarly be applied to the inner surface of tuft 570 of Figure 5A. Additional forms are contemplated where composition is applied as described heretofore with regard to Figures 3A and 3B.

Similarly, the tufts 572 shown in Figures 5E and 5F may similarly comprise composition sites 595. However, filled tufts 572, as explained hereafter, comprise a plurality of filaments which would typically inhibit composition from being applied to an inner surface of the filled tuft 572 - particularly the distal end 554 of these tufts. So, where the discontinuities of the secondary web 180 comprise filled tufts 572, the composition of compositions sites 595 may be positioned closer to the base 550 of the tufts 572.

With regard to the tunnel tufts 570, filled tufts 572, and outer tufts 530 oriented in the negative Z-direction, as shown in Figures 5C, 5D, 5G, and 5H, composition sites 590 may be disposed on the distal area 554, 555 of the tunnel tuft 570, filled tufts 572, or outer tuft 530 and on a portion of the side walls 556, 557 of the tunnel tuft 570, filled tufts 572, or outer tuft 530. The composition site 590 may be configured as described above with regard to Figures 5A, 5B, 5E, and 5F. As shown, specifically with regard to Figures 5C and 5D, the composition site 590 may be disposed on an inner surface of the tuft 570 as opposed to the outer surface depicted in Figures 5A and 5B. In such forms, where the secondary web 180 is utilized as a topsheet of a disposable absorbent article, the composition site 590 may comprise a hydrophilic composition which can improve the liquid acquisition time of the absorbent article. Forms of the present invention are contemplated where composition sites are provided on land areas 540 of the secondary web 180. In such forms, the composition in the land areas 540 may be the same or different than the compositions in the composition sites 590 and/or 595. Additional forms are contemplated where composition is applied as described heretofore with regard to Figures 3A and 3B.

Regarding Figures 5G and 5H, composition site 590 may be disposed on the distal ends 554, 555 of the filled tuft 572 and outer tuft 530 respectively. The composition site 590 may be disposed on the outer surface of the filled tuft 572 or the outer surface of the outer tuft 530. Additional composition sites 595 may be provided adjacent the base 550 of the filled tuft 572. As explained previously, the filaments of the filled tufts 572 would inhibit composition from being applied to the inner surface of the filled tuft 572 - particularly the distal end 554. In such forms, the composition provided in the composition sites 595 may be hydrophilic which can enhance fluid acquisition times where the secondary web 180 is utilized as a topsheet of a disposable absorbent article.

Regarding Figures 5A-5H, in some forms, composition site 590 may be provided in conjunction with the composition site 595 or the composition site 590 may be provided to the secondary web 180 sans the composition site 595 or vice versa. And, forms of the present invention are contemplated where a plurality of composition sites are provided to the secondary web 180 or the precursor web configured similarly to that of composition site 590. Forms of the present invention are contemplated where a plurality of composition sites are provided to the secondary web 180 or the precursor web configured similarly to that of composition site 595. Forms of the present invention are contemplated where a plurality of composition sites are provided to the land areas 540 of the secondary web 180. The plurality of composition sites in the land areas 540 may be provided in conjunction with the composition sites 590 and/or 595 or sans the compositions sites 590 and/or 595.

Figures 5A-5D illustrate tunnel tufts 570 which may be formed with nonwoven webs comprising extensible fibers or films. The tunnel tufts 570 and outer tufts 530 disclosed herein comprise a plurality of looped filaments that are substantially aligned such that each of the tunnel tufts 570 and outer tufts 530 have a distinct linear orientation and a longitudinal axis of the tuft, e.g. 570, 530. Another characteristic of the tunnel tufts 570 and outer tufts 530 shown in Figures 5A-5D - formed with extensible non-crimped fibers -- can be their generally open structure characterized by open void area 533 defined interiorly of the tunnel tuft 570. The term "void area" is not meant to refer to an area completely free of any fibers. Rather, the term is meant as a general description of the general appearance of tunnel tuft 570. Therefore, it may be that in some tunnel tufts 570 a non-looped filaments or a plurality of loose non-looped filaments may be present in the void area 533. By "open" void area is meant that the two longitudinal ends of tunnel tuft 570 are generally open and free of filaments, such that the tunnel tuft 570 can form something like a "tunnel" structure in an uncompressed state, as shown in Figures 5A-5D.

In contrast to the tunnel tufts 570 shown in Figures 5A-5D, secondary webs 180 of the present invention comprising crimped filament spunbond nonwoven layer(s) or strata may form filled tufts 572 (shown in Figures 5E-5H). Rather than being stretched during mechanical manipulation, crimped filament layers or strata simply uncurl. This leads to the filaments "filling" the void space of the tufts thereby forming filled tufts 572.

Where the secondary webs 180 of the present invention comprise crimped filaments, the secondary web 180 has a higher caliper for a given basis weight. This higher caliper can in turn deliver consumer benefits of comfort due to cushiony softness, faster absorbency due to higher permeability, and improved masking. Additional benefits may include less redmarking, higher breathability and resiliency.

### Nested Tufts

Yet another example of discontinuities which may be utilized in the present invention comprise nested tufts. Methods and apparatuses for making webs comprising nested tufts are described in U.S. Patent Publication Nos. US 2012/0064298 and 2016/0074252.

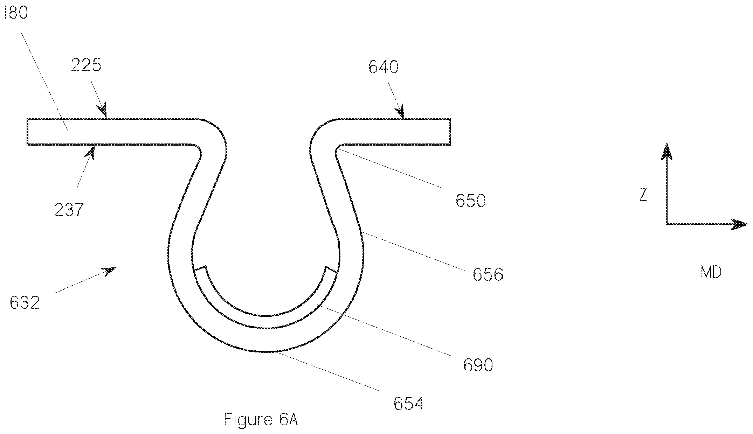

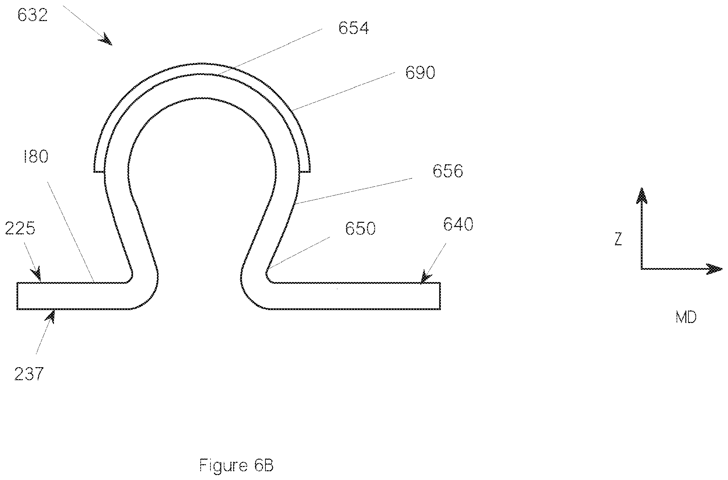

A schematic cross section of an exemplary nested tuft is shown in Figures 6A and 6B. As shown, the tuft 632 may be oriented in the negative Z-direction or the positive Z-direction. Similar to the tunnel tufts / filled tufts of Figures 5A-5H, the tufts 632 may comprise composition sites 690 disposed on a portion of an inner surface and/or outer surface of side walls 656 and distal end 654 of the tufts 632. In some forms, the composition site 690 may be disposed on the sidewalls 656 sans the distal end 654 or vice versa. In other forms, the composition sites 690 may be disposed on the side walls 656 and extend toward a base 650 of the nested tuft 632. Forms of the present invention are contemplated where composition is provided to the tuft 632 on an outer surface thereof in combination with the composition sites 690 or sans the composition sites 690. Forms of the present invention are contemplated where composition is provided on land areas 640 of the secondary web 180. The composition provided on the land areas 640 may be provided sans the composition provided in the composition site 690 or in conjunction therewith. In addition, compositions applied to the land areas 640 may be the same as composition(s) applied to the composition sites 690 or may be different.

For those forms of the present invention where the nested tufts 632 extend in the negative Z-direction (away from a user of a disposable absorbent article) - assuming the secondary web 180 is being used as a topsheet - the composition site 690 may comprise a hydrophilic composition. For those forms of the present invention where the nested tufts 632 extend in the positive Z-direction (toward the user of a disposable absorbent article) - assuming the secondary web 180 is being used as a topsheet - the composition site 690 may comprise a hydrophobic composition.

Where the nested tufts 632 are oriented in the negative Z-direction, forms of the present invention are contemplated where the composition is disposed on an inner surface of the sidewalls and/or inner surface of the distal end of the nested tufts. Such forms may be beneficial where the secondary web 180 forms a portion of a topsheet of a disposable absorbent article - with the distal ends 654 oriented toward an absorbent core of the disposable absorbent article - in that a hydrophilic composition on the inner surface can improve acquisition speeds of liquid insults. In contrast, if the hydrophilic composition were instead disposed on an outer surface, liquid insults may not have easy access to the hydrophilic composition which may negatively impact liquid acquisition speeds of the topsheet. This aspect may be particularly relevant where the constituent chemistry of the material of the secondary web 180 is hydrophobic.

Additionally, forms of the present invention are contemplated where, the secondary web 180 comprises multiple layers or multiple strata. The nested tufts 632 described herein may be formed in a secondary web comprising multiple layers or multiple strata.

Additional tufts are described in U.S. Patent Application Publication No. 2014/0121624.

### Corrugations

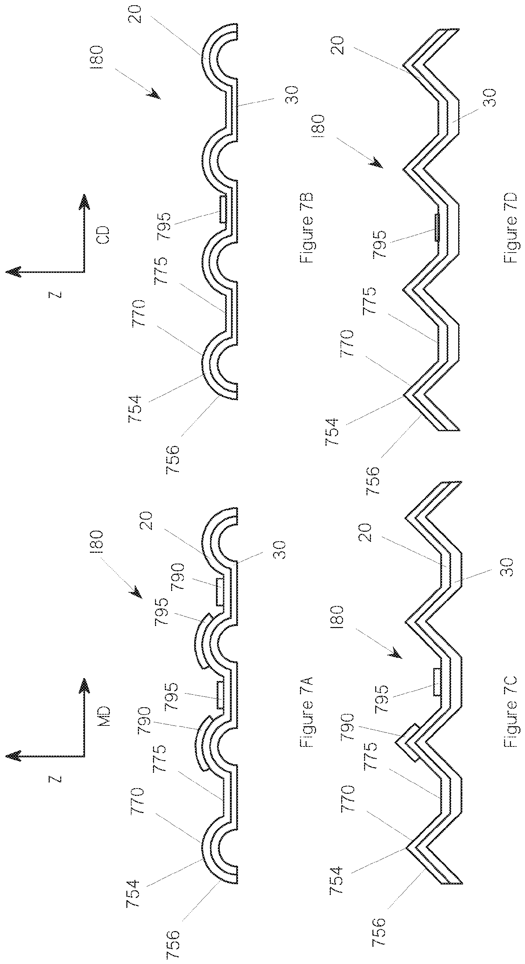

The discontinuities of the present invention may additionally comprise corrugations comprising ridges and grooves. Schematic cross sections of secondary webs comprising corrugations are provided with regard to Figures 7A-7D. Descriptions regarding the creation of corrugations can be found in U.S. Patent Nos. 6,458,447; 7,270,861; 8,502,013; 7,954,213; 7,625,363; 8,450,557; 7,741,235; US Patent Application Publication Nos. US2003/018741; US2009/0240222; US2012/0045620; US20120141742; US20120196091; US20120321839; US2013/0022784; US2013/0017370; US2013/013732; US2013/0165883; US2013/0158497; US2013/0280481; US2013/0184665; US2013/0178815; US2013/0236700; PCT Patent Application Publication Nos. WO2008/156075; WO2010/055699; WO2011/125893; WO2012/137553; WO2013/018846; WO2013/047890; and WO2013/157365.

As shown, the secondary web 180 of the present invention may comprise ridges 770 which can extend in a direction generally parallel to the MD or generally parallel to the CD. The ridges 770 may comprise any suitable shape. For example, as shown, the ridges 770 may have an arcuate shape. As another example, the ridges 770 may comprise a triangular shape. Regardless of the shape, the ridges 770 may comprise - similar to their tuft counterparts - a distal end 754 and sidewalls 756 extending from a groove 775. Additionally, examples are contemplated where a nonwoven web constructed in accordance with the present invention comprises at least one ridge having an arcuate shape and one ridge comprising a triangular shape.

As shown, the secondary web 180 may comprise composition sites 790 disposed on the distal ends 754 of the ridges 770. The composition sites 790 may also be disposed on at least a portion of the sidewalls 756. Forms are contemplated where the composition sites 790 are provided to only a portion of the distal ends 754 of the secondary web 180. Additional forms are contemplated where the composition sites 790 are provided to the distal ends 754 of all of the ridges 770 of the secondary web 180.

Additionally, in some forms of the present invention, composition sites 795 may be provided to the grooves 775 of the secondary web 180. In some forms, the composition sites 795 may be provided to each of the grooves between adjacent ridges 770. In some forms, the composition sites 795 may be provided to only a portion of the grooves 775 between adjacent ridges 770.

In some forms, the composition sites 790 may be provided sans the composition sites 795 and vice versa. Additionally, the composition provided in the composition sites 790 may be the same as or different from the composition provided in the composition sites 795. Additionally, forms of the present invention are contemplated where composition sites are provided on an inner surface of the ridges 770 or an inner-surface of the grooves 775.

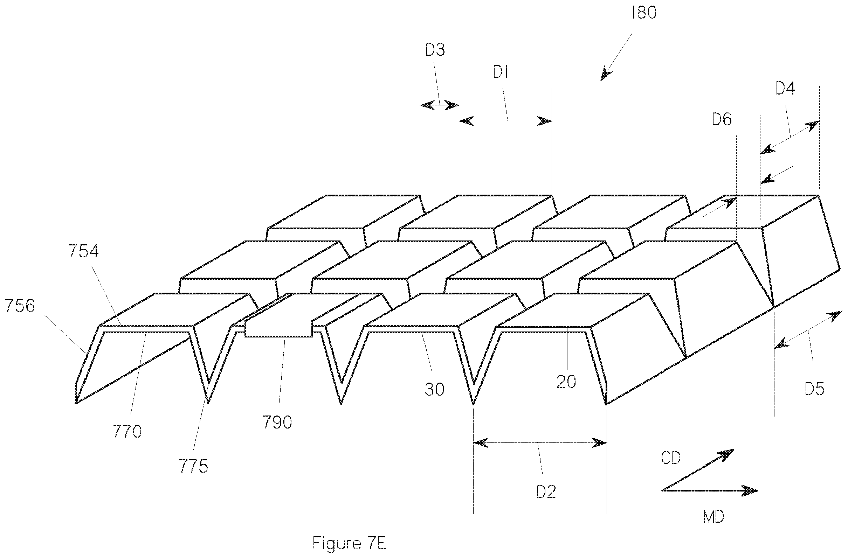

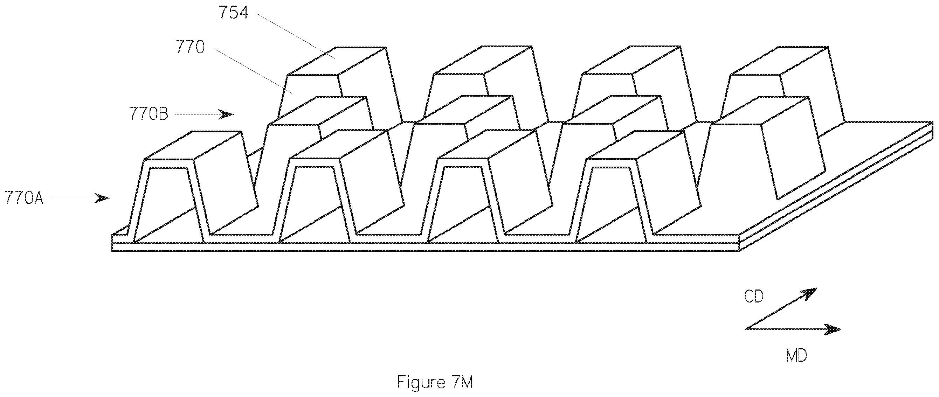

Additional forms of the present invention are contemplated where the secondary web 180 described with regard to Figures 7A-7D, is additionally processed such that corrugations are created in both an MD and CD direction or at angles thereto. As shown in Figure 7E, the secondary web 180 may be provided to a nip between intermeshing rolls having concave and convex patterns. The dimensions D1, D2, and D3 of the corrugations 770 correlate to the spacing of the concave / convex patterns on the rolls in the MD. The dimensions D4, D5, D6 correlate to the concave / convex patterns on the rolls in the CD. The density of the secondary web 180 at the side walls 756 can be changed by adjusting the depths and the like of the rolls as needed. Forms of the present invention are contemplated where the corrugations 770 alternate in adjacent rows as shown in Figure 7M. For example, corrugations 770 in a first row 770A may be offset from corrugations 770 in a second row 770B. Alternating corrugations 770 are further described in U.S. Patent No. 8,784,972. Additional forms are described in U.S. Patent No. 7,955,549.

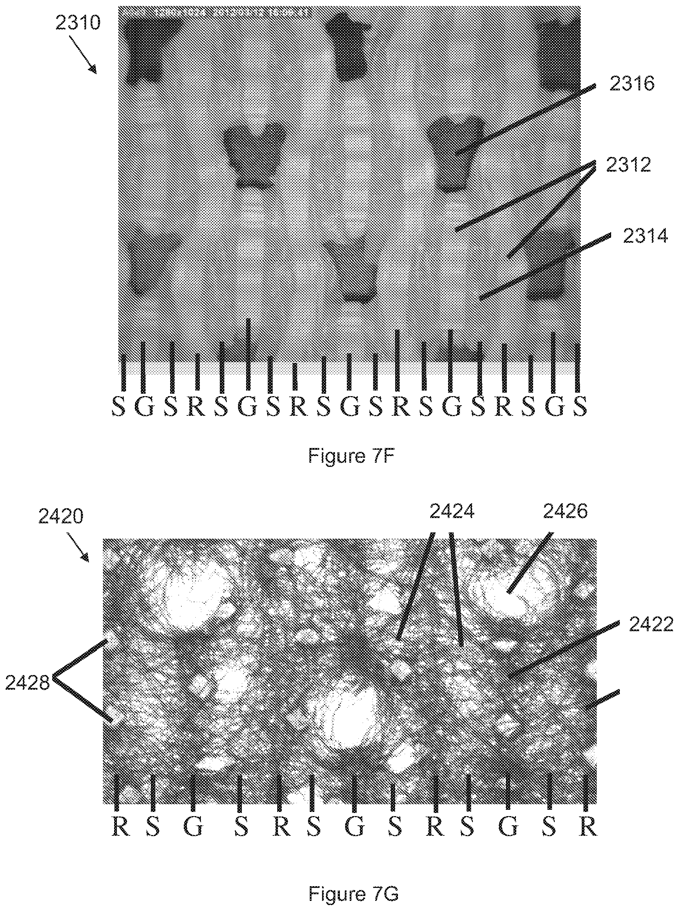

Another example of a secondary web 180 comprising corrugations is shown with regard to Figures 7F. Figure 7F is a top view of a 25 gsm polyethylene film web 2310 (film is stretched/flattened out to show high basis weight regions 2312 and low basis weight regions 2314). Web 2310 further shows ridges R (distal ends), grooves G, and sidewalls S. Apertures 2316 are present in the grooves G. As apparent, the high basis weight regions 2312 are located in the ridges R and grooves G, whereas the low basis weight regions 2314 are located in the sidewalls S.

In the case of a nonwoven, the basis weight is also decreased in the stretched areas, again resulting in a web with alternating regions of higher and lower basis weight, with the higher basis weight regions located in the tops of the ridges and bottoms of the grooves, and the lower basis weight regions located in the sidewalls in-between. Figure 7G is a top view of a 60 gsm polypropylene nonwoven web 2420 (nonwoven is stretched/flattened out to show high basis weight regions 2422, and low basis weight regions 2424). Web 2420 further shows ridges R, grooves G, and sidewalls S. Apertures 2426 are present in the grooves G. Thermal or fusion bond points 2428 may be present in various locations on the web 2420. As apparent, the high basis weight regions 2422 are located in the ridges R and grooves G, whereas the low basis weight regions 2424 are located in the sidewalls S. In the case of a nonwoven, the web thickness may not decrease in the stretched regions because the fibers may detangle and move away from each other. However, the thickness of some of the individual fibers may decrease as a result of the stretching. Note that the "regions" of the web used to characterize basis weight exclude the apertures themselves.

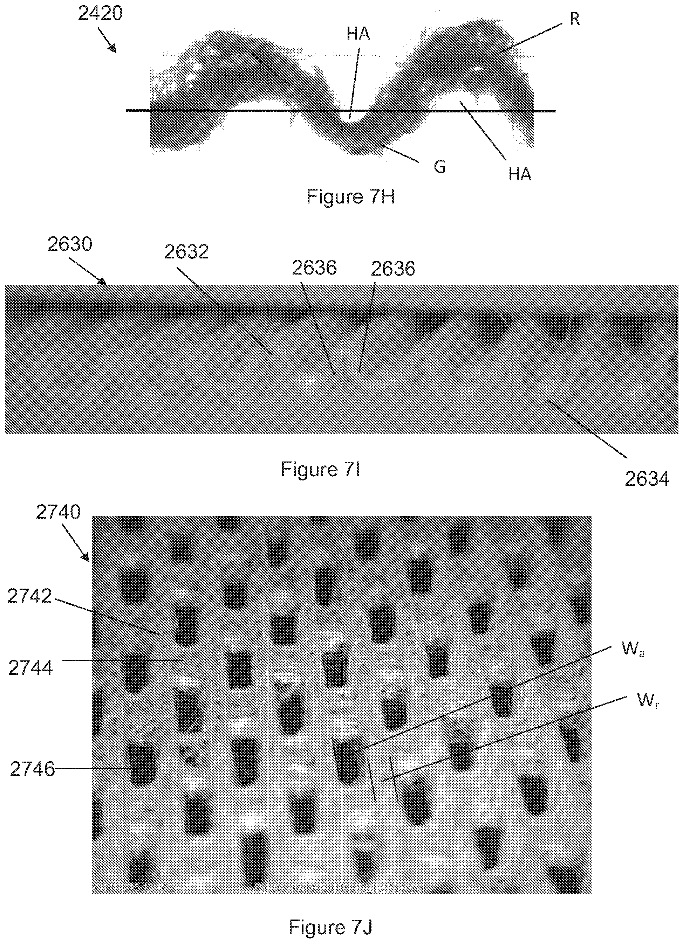

Figure 7H is a cross-section view of the web 2420 shown in Figure 7G showing ridges R, grooves G, and axis X drawn horizontally through a cross-section of the web; the area above the X axis but under the top of the ridge is hollow, or comprises a hollow area HA. Likewise, the area below the X axis but above the bottom of the groove is hollow, or comprises a hollow area HA. Suitably, the web thickness at the tops of the ridges and the web thickness at the bottoms of the grooves are similar. The web thickness at the tops of the ridges and the web thickness at the bottoms of the grooves may be similar to the web thickness at the sidewalls. By similar, it is meant that the thicknesses are within about 60% of one another. Or, the web thickness at the tops of the ridges and the web thickness at the bottoms of the grooves is greater than the web thickness at the sidewalls. Figure 7I is side perspective view of another nonwoven web 2630 having ridges 2632, grooves 2634, and sidewalls 2636. Figure 7J is a top perspective view of 28 gsm polyethylene/polypropylene bico nonwoven web 2740 comprising ridges 2742 and grooves 2744 and apertures 2746 wherein the aperture width Wₐ is greater than the ridge width Wᵣ.

Webs made by the processes and apparatuses described herein may comprise ridges that run discontinuously across a deformed zone, or, ridges that run continuously across a deformed zone. To create such apertured web materials, the rolls used may comprise zones of ridges and grooves. Or, the rolls can have zones where the ridges are different heights, thereby creating differing depth of engagement (DOE), differing depth below the raised ridge, and thus apertures with differing widths and open areas. Alternatively or in addition, the rolls may comprise different zones, wherein ridge heights are different in different zones.

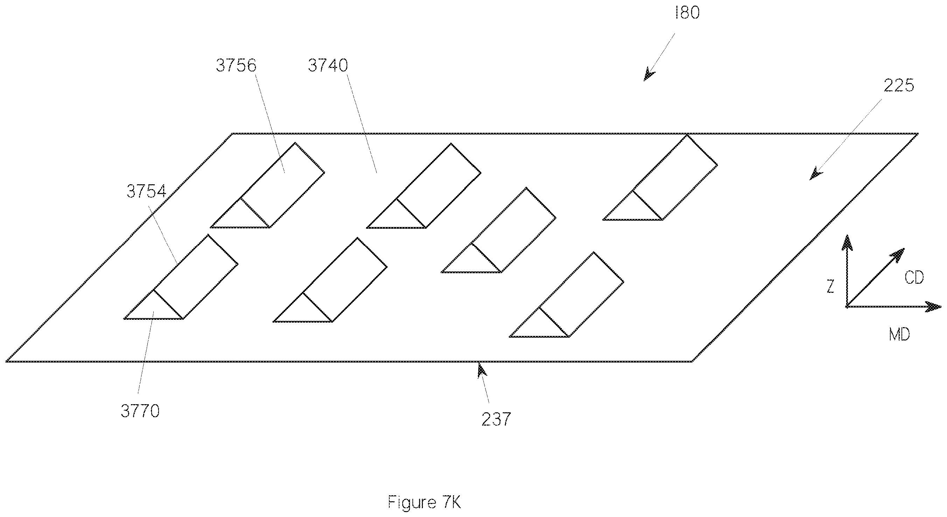

Still in other forms of the present invention, the secondary web 180 may comprise rib like elements 3770 (corrugations) shown in Figure 7K. The corrugations 3770 comprise a major axis and a minor axis defining an elongated cubical, ellipsoidal or other similar rib-like shape. The major axis and the minor axis of the corrugations 3770 may each be linear, curvilinear or a combination of linear and curvilinear. Each of the corrugations 3770 comprises a distal end 3754 and sidewalls 3756 extending from the generally planar first surface 225. Forms of the present invention are contemplated where the material web 100 comprises an undeformed first region 3740 (land areas).

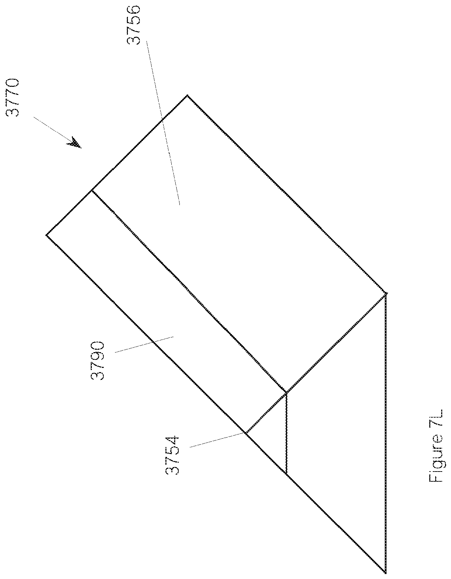

Referring now to Figures 7K and 7L, the first and second regions of the secondary web 180 may be formed from a precursor web that is substantially planar. Said starting precursor web can be fed through an apparatus which forms the corrugations 3770 on the precursor web in predefined areas resulting in corrugated second regions of the secondary web 180 and undeformed regions 3740 of the secondary web 180. Processes for forming the corrugations 3770 are discussed in additional detail in U.S. Patent Application Publication No. 2004/0137200.

For the forms of the invention described in Figures 7J-7L, composition sites may be disposed on the distal ends of the ridges / corrugations and/or in the grooves between adjacent ridges / corrugations. For those forms where ridges form a wearer-facing surface of an absorbent article, the composition disposed on the distal ends may be hydrophobic which can provide good masking of liquid insults. Additionally, a composition disposed in the grooves - if any - may be hydrophilic which can enhance the liquid acquisition of the secondary web 180. For those forms where ridges are oriented away from a wearer, hydrophilic composition sites may be provided on an inner surface of the ridges while hydrophobic composition may be provided to the grooves. Forms of the present invention are contemplated where a composition is provided on at least a portion of the distal ends of the ridges / corrugations sans a composition on at least a portion of the grooves between adjacent ridges / corrugations or vice versa.

### Fusion Bonds

Still another exemplary process which may be utilized as a first unit operation 140 (shown in Figure 2) is a process that can provide fusion bonding to an absorbent article. The distinction between embossments (discussed with regard to Figures 4A and 4B) and fusion bonding is that generally, embossing does not result in the fusion of layers.

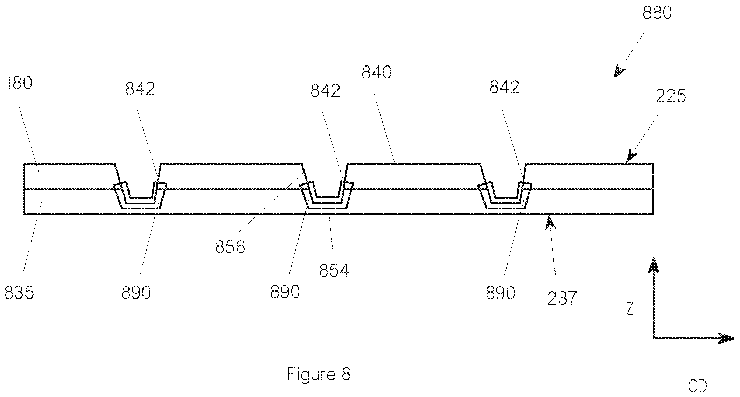

As shown in Figure 8, a laminate 880 comprising the secondary web 180 and a second substrate 835. As shown, the laminate 880 may comprise a plurality of discrete bond sites 842 which bond the secondary web 180 to the second substrate 835. As shown, composition sites 890 may be disposed at distal ends 854 of the bond sites 842. Additional forms of the present invention are contemplated where composition sites are provided in land areas 840. In some forms, a hydrophobic composition may be provided in the composition sites 890. The hydrophobic composition may preclude the buildup of liquid insult in the bond sites 842. In such forms, the composition may be disposed only over a portion of the sidewalls of the bond sites 842. As such, the hydrophobic composition can encourage liquid to escape the bond site 842 via portions of the sidewall more proximal to the land area 840. Forms of the present invention are contemplated where the compositions sites 890 are provided on the secondary web 180 sans the composition sites disposed on the land areas 840 and vice versa.

Processes and apparatuses for forming fusion bonds in laminate structures is provided in additional detail in U.S. Patent Application Publication No. 2015/0173961.

### Additional Structures

The discontinuities described herein may be utilized in any suitable combination with one another. For example, a secondary web may comprise tunnel tufts, nested tufts, outer tufts, embossments, and/or fusion bonds. As another example, a secondary web may comprise filled tufts, outer tufts, embossments and/or fusion bonds. As yet another example, a secondary web may comprise corrugations, embossments, and/or fusion bonds.

Additionally, the precursor or secondary webs described herein may comprise apertures which can enhance fluid acquisition times. The apertures may be utilized in conjunction with any of the discontinuities described herein or any combination of discontinuities described herein.

The apertures may be produced by any suitable method. Some suitable methods include stretch aperturing as described in U.S. Patent No. 5,658,639; 5,628,097; 5,916,661; 7,917,985; and U.S. Patent Application Publication No. 2003/0021951. Additional processes are described in U.S. Patent Nos. 8,679,391 and 8,158,043, and U.S. Patent Application Publication Nos. 2001/0024940 and 2012/0282436. Other methods for aperturing webs are provided in U.S. Patent Nos. 3,566,726; 4,634,440; and 4,780,352.

The apertures may be arranged in any suitable manner. In some forms, the apertures may be arranged such that the apertures form indicia on an absorbent article. Exemplary aperture arrays / patterns are disclosed in U.S. Patent Application Publication No. 2016/0129661. Additionally, forms are contemplated where the precursor web and/or secondary web comprises apertures which are treated with a composition, e.g. a surfactant. Such treatment can facilitate fluid acquisition by the apertures. Treatment of apertures via non-contact printing methods are disclosed in U.S. Patent Application Serial No. 62/291709.

Additional forms of the present invention are contemplated where the discontinuities form a shaped web. Shaped webs are a shaped nonwoven fabric directly formed on a shaped forming belt with continuous spunbond filaments in a single forming process. The web of the present disclosure can assume a shape which corresponds to the shape of the forming belt.

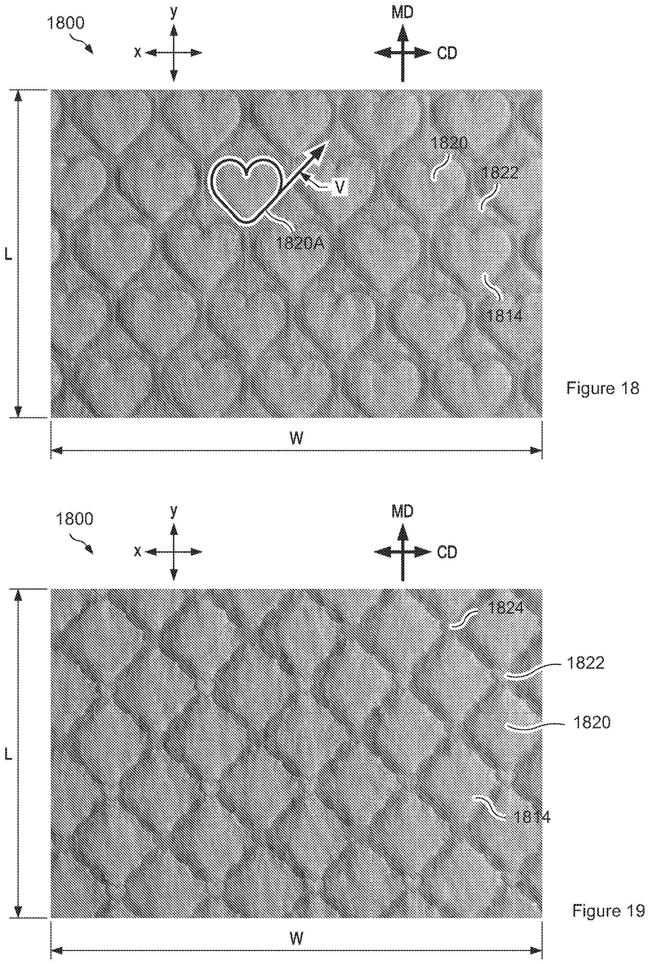

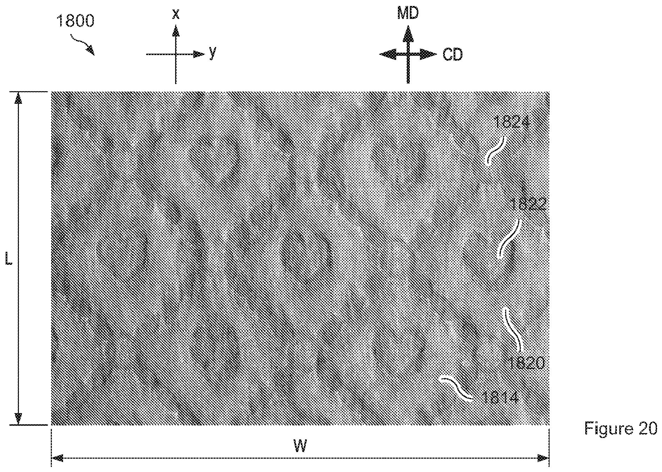

As shown in Figures 18-20, a second surface 1814 of a web 1800 is shown. The three-dimensional features of the web 1800 are formed by spinning fibers directly onto a forming belt having a pattern of corresponding three-dimensional features. In one sense the web 1800 is molded onto a forming belt that determines the shapes of the three-dimensional features of the web 1800. The web 1800 shown may comprise a first three-dimensional feature 1820 that is heart shaped, and as indicated as one exemplary first three-dimensional feature 1820A is defined by a curvilinear closed heart-shaped element. A curvilinear element can be understood as a linear element having at any point along its length a tangential vector V, with the closed shape being such that the tangential vector V has both MD and CD components that change values over greater than 50% of the length of the linear element of the closed figure. It is worth noting that the three-dimensional features described can be any suitable shape.

Importantly, the web 1800, in addition to taking the shape of the forming belt, because of the attributes of the forming belt and the apparatus for forming the fabric is imparted with beneficial properties for use in personal care articles, garments, medical products, and cleaning products. Specifically, because of the nature of the forming belt and other apparatus elements, as described below, the three-dimensional features of the web 1800 have intensive properties that can differ from feature to feature in ways that provide for beneficial properties of the web 1800 when used in personal care articles, garments, medical products, and cleaning products. For example, first three-dimensional feature 1820 can have a basis weight or density that is different from the basis weight or density of second three-dimensional feature 1822, and both can have a basis weight or density that is different from that of third three-dimensional feature 1824, providing for beneficial aesthetic and functional properties related to fluid acquisition, distribution and/or absorption in diapers or sanitary napkins.

Forms of the present invention are contemplated where composition is provided on at least a portion of the first three-dimensional feature 1820, at least a portion of the second three-dimensional features 1822 and/or at least a portion of the third three-dimensional features 1824. In some forms, the composition provided on the first three-dimensional features 1820 may be more hydrophobic than the composition provided to the third three-dimensional features 1824. In some forms, the composition provided to the first three-dimensional feature 1820 may be more hydrophilic than the composition provided to the third three-dimensional features 1824. In such forms, the composition provided to the third three-dimensional features 1824 may be a blood modifying agent.

Additional details regarding shaped webs 1800 and processes of making shaped webs 1800 is discussed in additional detail in U.S. Patent Application Serial No. 15/221624.

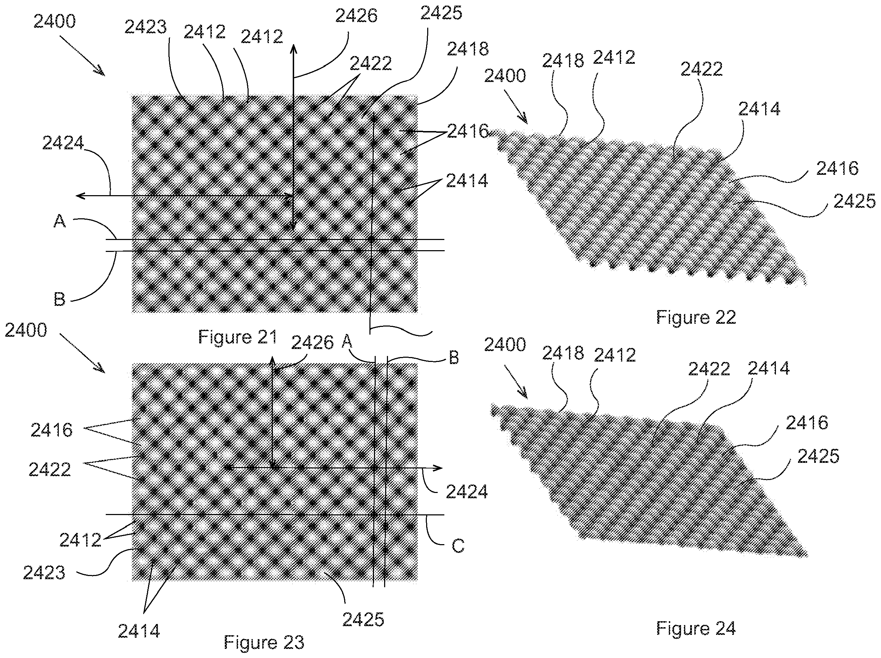

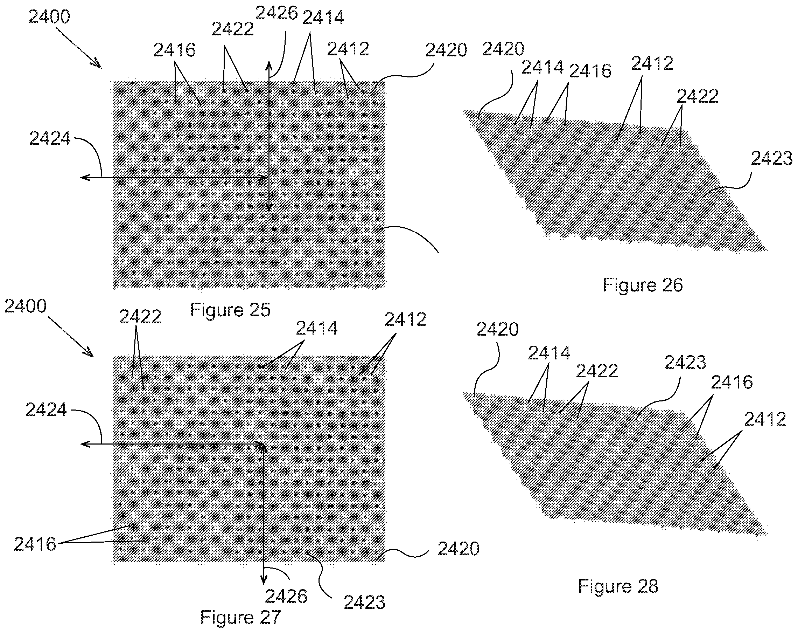

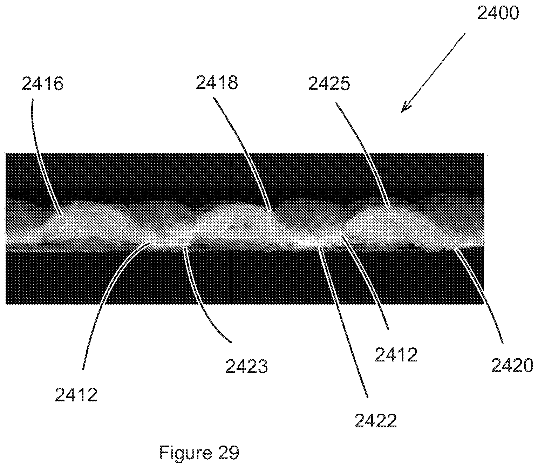

Referring generally to Figure 21-29, a liquid permeable substrate 2400 may comprise a plurality of land areas 2412, a plurality of recesses 2414, and a plurality of projections 2416. The plurality of projections 2416 may form the first elements having the first z-directional height, and the land areas 2412 may form the second elements having the second z-direction height. The plurality of land areas 2412, the plurality of recesses 2414, and the plurality of projections 2416 may together form a first three-dimensional surface on a first side 2418 of the substrate 2400. The plurality of land areas 2412, the plurality of recesses 2414, and the plurality of projections 2416 may also form a second three-dimensional surface on a second side 2420 of the substrate 2400. The projections 2416 may be generally dome shaped on a wearer-facing surface of the liquid permeable substrate 2400 and may be hollow arch-shaped on the garment-facing surface of the substrate 2400. All of, or a majority of (i.e., more than 50% of, or more than 75% of), or substantially all of, the recesses 2414 may define an aperture 2422 therein at a location most distal from a top peak 2425 of an adjacent projection 2416. A perimeter 2423 of a majority of, or all of, the apertures 2422 may form a bottommost portion or plane of the substrate 2400, while the top peak 2425 (i.e., uppermost portion) of a majority of, or all of, the projections 2416 may form a topmost portion or plane of the substrate 2400. In other instances, the substrate may not have apertures within the recesses 2414 and the portion of the recesses 2414 most distal from the top peaks 2425 of the projections 2416 may form the bottommost portion or plane of the substrate 2400. The apertures 2422 may extend through the first and the second layers of the substrate 2400.

The land areas 2412 may be positioned intermediate: (1) adjacent projections 2416, (2) adjacent recesses 2414 and/or adjacent apertures 2422. The land areas 2412 may also surround at least a portion of, or all of, a majority of, or all of, the recesses 2414 and/or the apertures and at least a majority of, or all of, the projections 2416. The land areas 2412 may be positioned between a plane of a perimeter of at least a majority of the apertures 2422 and a plane of at least a majority of the top peaks 2425 of the projections 2416.

Forms of the present invention are contemplated where webs of the present disclosure comprise compositions applied to a plurality of the projections 2416, a plurality of the land areas 2412, a plurality of recesses 2414, and/or a plurality of apertures 2422. For example, where the plurality of projections are facing in the positive Z-direction and form a portion of a topsheet of a disposable absorbent article, a hydrophobic composition may be applied to distal ends of a plurality of the projections 2416. In conjunction therewith, or independently therefrom, a composition may be applied to a plurality of land areas 2412. In such forms, the composition may be hydrophilic. In conjunction with the foregoing, or independently therefrom, a composition may be applied to the land areas 2414 and/or the apertures 2422. In such forms, the composition may be hydrophilic. In some forms, the composition may be a hydrophobic blood modifying agent as disclosed herein.

The projections 2416, land areas 2412, recesses 2414, and apertures 2422 are described in additional detail along with description on how to make such webs in U.S. Patent Application Publication Nos. 2015/0250662A1 and 2015/0250663A1.

The projections 2416 may be configured as described heretofore with regard to Figures 3A and 3B. Similarly, the application of composition(s) to the projections 2416, land areas 2412, recesses 2414, and apertures may be as described herein with regard to the webs described herein.

### Additional Processes

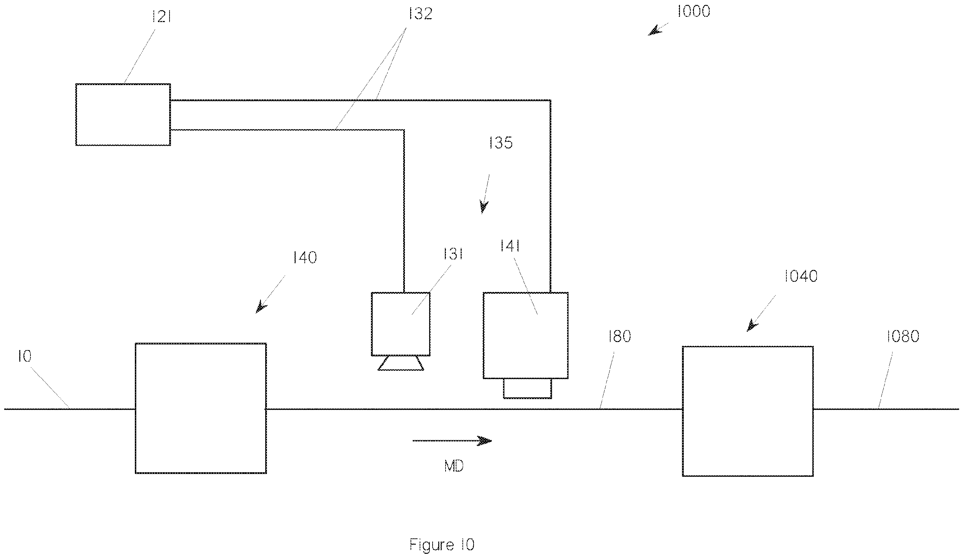

Forms of the present invention are contemplated where the apparatus 100 of Figure 10 further comprises a second unit operation 1040. As shown, the secondary web 180 may be provided to the second unit operation 1040. Recall, that the secondary web 180 comprises a first plurality of discontinuities. The second unit operation 1040 may provide the secondary web 180 with a second plurality of discontinuities thereby creating a tertiary web 1080.

Still referring to Figure 10, as shown, the printer 141 and camera 131 may be positioned downstream of the first unit operation 140 such that a first plurality of composition sites may be provided to the secondary web 180 associated with the first plurality of discontinuities. Additionally, the printer 141 may deposit a second plurality of composition sites which are associated with the second plurality of discontinuities on the tertiary web 1080. In contrast, forms of the present invention are contemplated where the printer 141 is disposed upstream of the first unit operation 140 (similar to what is shown in Figure 1B). Additional forms are contemplated where the printer 141 is disposed downstream of the second unit operation 1040. Similarly, forms are contemplated where the camera 131 is positioned as described heretofore with regard to Figures 1A and 1B or any discussion pertinent thereto. The tertiary web 1080 may comprise any suitable combination of discontinuities as described herein.

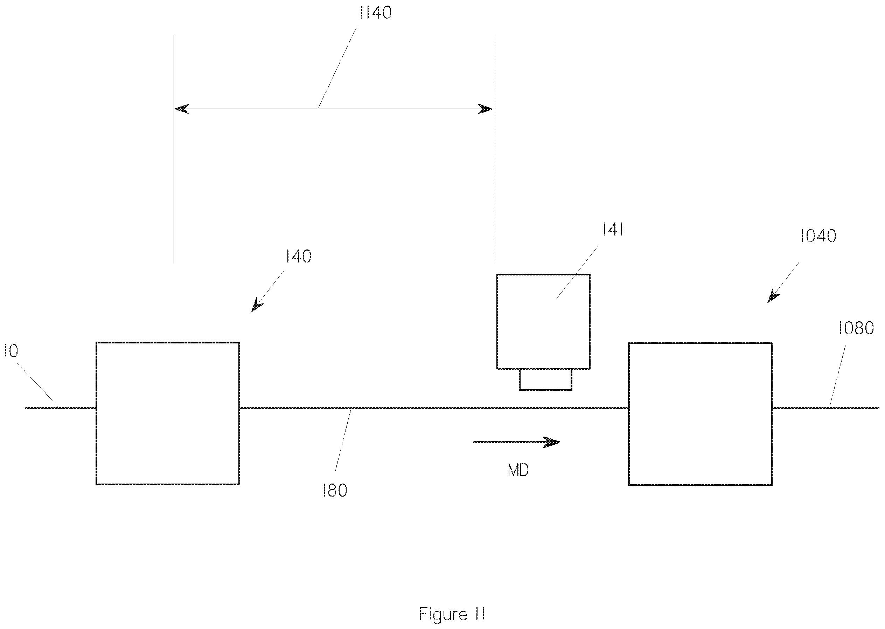

Additional processes are contemplated which do not utilize a visual system. Examples are provided with regard to Figure 11. In some forms of the present invention, compositions may be associated with discontinuities without the use of a vision system. For example, as shown in Figure 11A, the printer 141 may be disposed between the first unit operation 140 and the second unit operation 1040. If the printer 141 is positioned within a distance 1140 between the first unit operation 140 and the printer 141, the secondary web 180 may not track to such an extent that a vision system is needed. In such forms, the step of forming the discontinuities and printing the first plurality of composition sites is simply a matter of sequencing. The same holds true whether the printer 141 is upstream or downstream of the first unit operation 140. For those forms where the printer 141 is positioned downstream of the second unit operation 1040, the printer 141 should still be within the distance 1140 to the first unit operation 140.

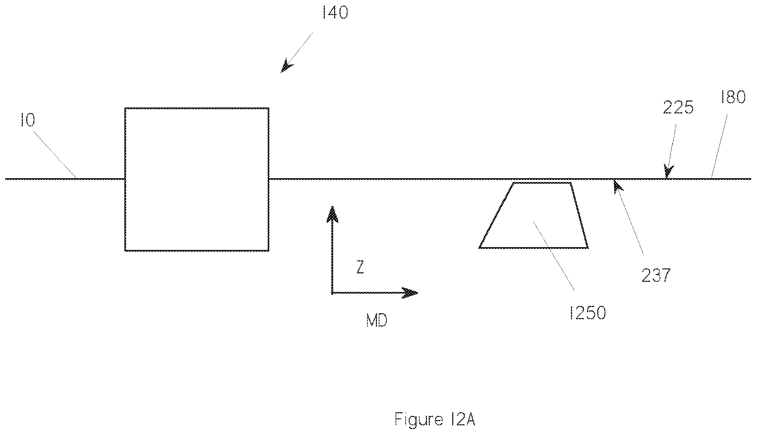

Heretofore, the application of composition(s) to the precursor web, secondary web, and/or tertiary web have been via a printer, i.e. non-contact deposition. However, forms of the present invention are contemplated where contact methods may be utilized. For example, a slot gun may be utilized to deposit composition(s) on the precursor, secondary, and / or tertiary web. As shown in Figure 12A, a nozzle 1250 may provide composition to the secondary web 180 post formation of the discontinuities. Where discrete composition sites are desired as described herein, the discontinuities can face in the negative Z-direction in the form shown. In such forms, as the secondary web 180 moves across the nozzle 840, the discontinuities contact the composition extruded through the nozzle 1250 as opposed to the entire secondary web 180. In such forms, composition may be deposited on the distal ends and/or sidewalls of the discontinuities while the land areas of the secondary web 180 remain without composition.

For the deposition of discrete composition areas, particularly on the distal ends of the discontinuities, the secondary web 180 may require tensioning upstream and downstream of the nozzle 1250 to minimize Z-direction movement of the secondary web 180. Additionally, depending on the height of the discontinuity and the viscosity of the composition being extruded through the nozzle 1250, the inlet / outlet angle of the secondary web 180 with respect to the nozzle 1250 may need to be adjusted.

For those forms where the second surface 237 of the secondary web 180 is desired to comprise a plurality of composition sites, where interconnectedness of the composition sites is preferred, the discontinuities of the secondary web 180 may be oriented in the positive Z-direction. In such forms, composition extruded by the nozzle 1250 would be deposited on the second surface 237 of the secondary web 180 sans the openings in the second surface 237.

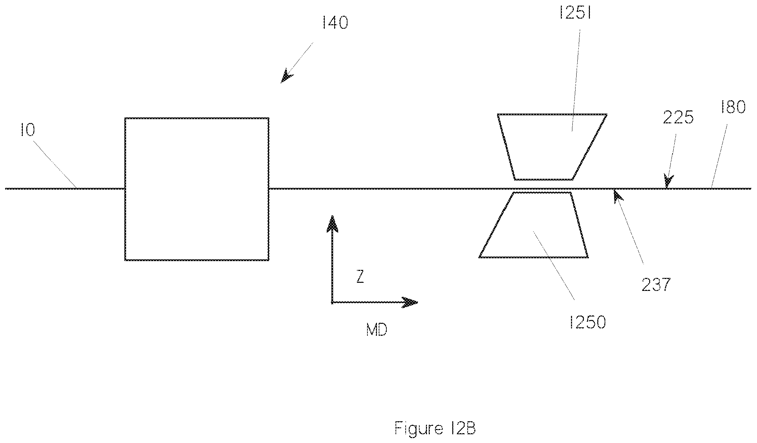

In some forms, as shown in Figure 12B, a second nozzle 1251 may be utilized to deposit composition on the secondary web 180. In such forms, assuming the discontinuities are facing in the positive Z-direction, the first nozzle 1250 may deposit composition on the second surface 237 sans the openings in the second surface 237, while the second nozzle 1251 deposits a second composition on the distal ends and/or sidewalls of the discontinuities.

Forms of the present invention are contemplated where one nozzle may comprise multiple slots from which compositions can be extruded. In such forms, one nozzle may deposit a plurality of compositions on the secondary web 180. For example, a nozzle may deposit a first composition in a center zone of the secondary web 180 and deposit a second composition in a second and/or third zone spaced from one another in the CD via the central zone. In some forms, the first composition may be different than the second composition. In other forms, a third composition may be deposited on the third zone. In such forms, the third composition may be different than the second composition and the first composition.

In general, application of compositions via a slot gun can allow for much greater basis weight of composition deposition than non-contact printing. However, application of compositions via slot gun do not allow for precision application as described above with regard to non-contact printing. Slot guns may similarly be utilized to deposit composition(s) on the tertiary webs 1080 (shown in Figure 11) independently or in conjunction with secondary webs 180.

The slot gun device is disclosed in additional detail in U.S. Patent Application No. 10/405,456 titled "Method and Device for Applying Fluids to Substrates" filed April 2, 2003 by Lippelt and assigned to Nordson Corp. Versions of slot gun device are commercially available as Meltex Series Model No. EP-11 and EP-12 slot guns from Nordson Corp., Duluth, Georgia.

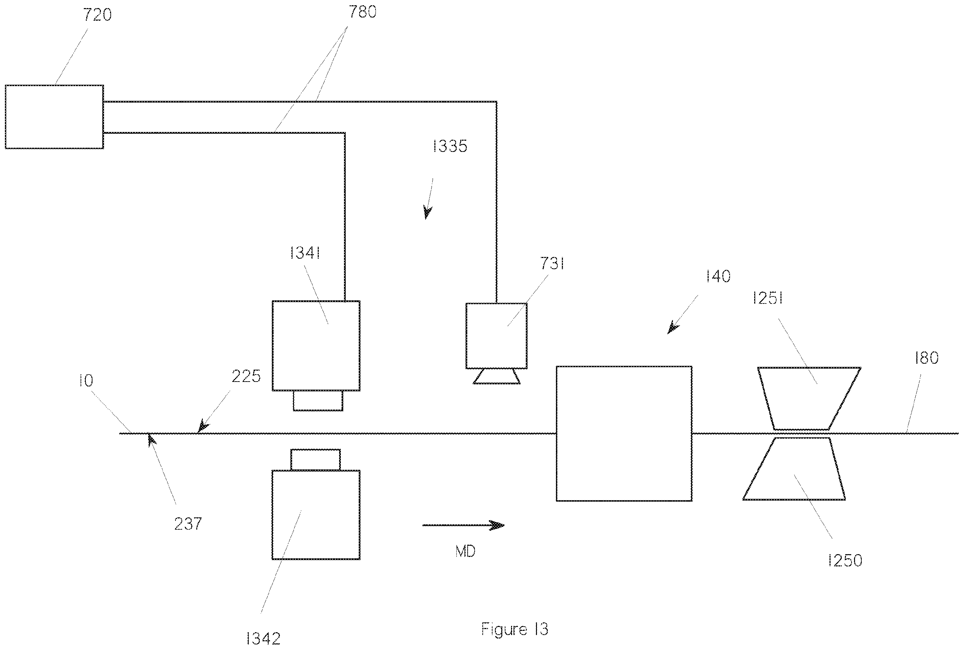

Still other forms of the present invention are contemplated where a combination of non-contact printing and contact deposition are utilized. For example, as shown in Figure 13, a printer 1341 may deposit a plurality of composition sites on the first surface 225 of the precursor web 10 while a second printer 1342 may deposit a plurality of composition sites on the second surface 237 of the precursor web 10. Where the first unit operation 140 is appropriately sequenced, the composition deposited by the first printer 1341 or the second printer 1342 may be disposed on an inner surface of the discontinuities formed by the first unit operation 140. Post formation of the discontinuities, the slot gun 1250 or 1251 can deposit composition on an outer surface of the discontinuities.

The slot guns 1250 and 1251 may be positioned in any suitable location. The printers 1341 and 1342 may similarly be configured / arranged in any suitable configuration as described herein.

### Periodicity of Discontinuities

In some forms of the present invention, the printer 141 (shown in Figures 1A and 1B) may deposit a first plurality of composiion sites based upon a first group of discontinuities 111 in accordance with a first stored pre-rendered pattern. The printer 141 may also deposit a second plurality of composition sites based upon a second group of discontinuities in accordance with a second stored pre-rendered pattern. In some forms, the first stored pre-rendered pattern and the second stored pre-rendered pattern may be based upon different periodicity and/or different phase shifts of the discontinuities. The determination of the periodicity of discontinuities is discussed in additional detail in U.S. Patent Application Serial No. 62/291709.

### Precursor Web

As discussed previously, the precursor web may comprise a single layer or multiple layers of material. For example, the precursor web may comprise a nonwoven layer. As another example, the precursor web may comprise a film layer. Still in other examples, the precursor web may comprise a laminate which includes multiple nonwoven layers, multiple film layers, or a combination thereof. In some forms, the precursor web may comprise a single layer with multiple stratums of material wherein the stratums of material are produced via a spunmelt process, e.g. spunbonding, spunlaying, or meltblowing.

The precursor web may comprise any suitable material. Some suitable examples include nonwovens, wovens, cellulosic materials, films, elastic materials, non-elastic materials, high-loft materials, and/or foams. The precursor webs may also comprise one or more layers of one or more nonwoven materials, one or more films, combinations of different nonwoven materials, combinations of different films, combinations of one or more films and one or more nonwoven materials, or combinations of one or more different materials, for example. Precursor webs having one or more layers of the same or similar materials are also within the scope of the present disclosure.

Precursor webs may comprise any suitable material. For example, precursor web materials may comprise PE/PP bi-component fiber spunbond webs. Other suitable precursor webs may comprise spunbond webs comprising side-by-side crimped fibers (e.g. PE/PP or PP/PP) that are bonded via calendar (thermal point) bonding or through-air bonding. For those configurations with multiple layers a first layer and second layer of the patterned apertured web of the present invention may comprise a crimped spunbond layer. For these configurations, the crimped spunbond layers may be combined from roll stock and joined as provided herein. However, where the precursor web comprises a first substrate and a second substrate, each may be crimped spunbond substrates formed on a spunbond manufacturing line where the first substrate is formed from a first spin beam while the second substrate is formed from a second spin beam.

Other suitable precursor webs may comprise carded staple fibers comprising polypropylene, polyethylene terephthalate, polyethylene / polypropylene bi-component, polyethylene / polyethylene terephthalate bi-component, or the like, which are calendar bonded, through-air bonded, resin bonded or hydroentangled. The precursor webs may comprise microfibers and/or nanofibers, optionally with other fibers. In some circumstances, multiple layer webs may be desired over a single layer webs (even at the same basis weight) due to increased uniformity/opacity and the ability to combine webs having different properties. For example, an extensible spunbond nonwoven carrier layer may be combined with a soft, crimped fiber nonwoven (spunbond or carded). The substrates may have the same or different surface energy, for example, the top layer may be hydrophobic and the lower layer may be hydrophilic. The layers may have different permeability/capillarity, e.g. the upper layer may have higher permeability and the lower layer have higher capillarity in order to set up a capillary gradient and aid in moving fluid away from the surface (or topsheet) of an absorbent article and into an absorbent core of the absorbent article.

Additionally, the precursor webs may comprise a surface treatment and/or additive to the constituent material of the precursor web. For example, the precursor web may comprise a hydrophobic surface treatment. For such webs, a composition applied in a composition site may be hydrophilic. Still in other examples, the precursor web may comprise a hydrophilic surface treatment or the constituent material of the precursor web may comprise hydrophilic material. For such webs, a composition applied in a composition site may be hydrophobic. As another example, precursor webs of the present invention may comprise a melt additive. In one specific example, the precursor web may comprise fibers which comprise a hydrophobic melt additive. In such example, at least one of the composition sites may comprise a hydrophilic composition.

Suitable melt additives and surface treatments of materials is discussed in additional detail in U.S. Patent Nos. 8,178,748, 8,026,188; 4,578,414; 5,969,026; U.S Patent Application Publication Nos. 2012/0100772; 2014/0272261; 2012/0296036; 2014/0087941; U.S. Patent Application Serial Nos. 14/849,630; 13/833,390; European Patent No. 2411061; and PCT Patent Application Publication No. 2012/162130.

Other suitable materials for precursor webs include films. Some suitable films are described in U.S. Patent Nos. 3,929,135; 4,324,426; 4,324,314; 4,629,643; 4,463,045; and 5,006,394.

### Compositions / Composition Sites

As mentioned previously, webs of the present invention may comprise a plurality of composition sites each of which comprises a composition. Similarly, the stored pre-rendered patterns described herein may correspond to a plurality of composition sites each of which comprises a composition. The composition sites on the webs described herein may comprise a variety of compositions. For example, a first plurality of composition sites may comprise a hydrophilic composition while a second plurality of composition sites may comprise a hydrophobic composition. And, as noted previously, some webs may comprise the first plurality of composition sites sans the second plurality of composition sites or vice versa. As noted herein, a third plurality of composition sites may be applied to a web in some forms. The third plurality of composition sites may be in addition to or sans the first plurality of composition sites and/or the second plurality of composition sites. Additional composition sites may be provided on a web.

As shown in Figures 3A and 3B the composition sites described herein may be applied to the precursor web, secondary web, and/or tertiary web in an array of discrete sites and/or in a plurality of arrangements. Forms of the present invention are contemplated where the composition sites applied to a web may be in the form of a plurality of stripes. And, while the plurality of stripes may be discrete from one another, forms are contemplated where the plurality of stripes are, at least in part, interconnected with one another. In such forms, each of the plurality of stripes may be registered with a discontinuity or may partially overlap a discontinuity or be offset from a discontinuity.

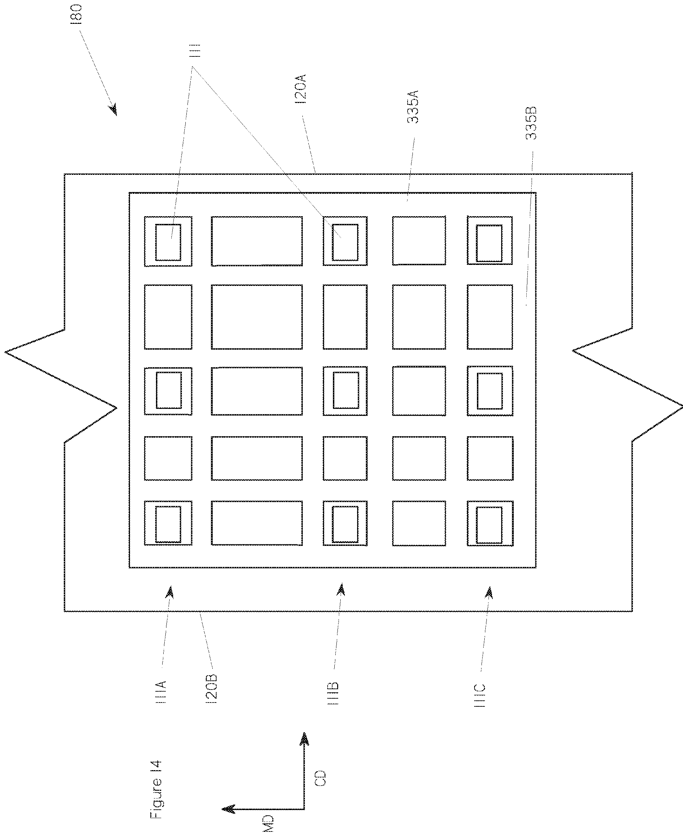

As shown in Figure 14, composition may be applied to the secondary web 180 in a plurality of stripes 335A and 335B which extend in the MD and CD, respectively. The plurality of stripes may be connected with one another forming a grid. As shown, the grid, at least in part, may surround a plurality of discontinuities 111. Forms are contemplated where the plurality of stripes 335A and 335B surround each of the plurality of discontinuities individually. Forms are contemplated where the plurality of stripes 335A and 335B surround a group, e.g. first group 111A, of the plurality of discontinuities. Forms are contemplated where the plurality of stripes 335A and 335B surround multiple groups of the plurality of discontinuities, e.g. first group 111A and second group 111B.

Still referring to Figure 14, the stripes 335A and 335B may be generally parallel with the MD and/or CD, respectively. In some forms, a first plurality of stripes may be generally parallel with the MD while a second plurality of stripes may be angled with respect to the MD and/or CD. In some forms, a first plurality of stripes may be generally parallel with the CD while a second plurality of stripes may be angled with respect to the CD. In some forms, a first plurality of stripes and a second plurality of stripes may each be angled with respect to the MD and CD. In such forms, the first plurality of stripes and the second plurality of stripes may interconnect with one another to form a diamond pattern. The diamond pattern may surround at least a portion of the plurality of discontinuities, may partially overlap at least a portion of the plurality of discontinuities, and/or may be registered with at least a portion of the plurality of discontinuities.

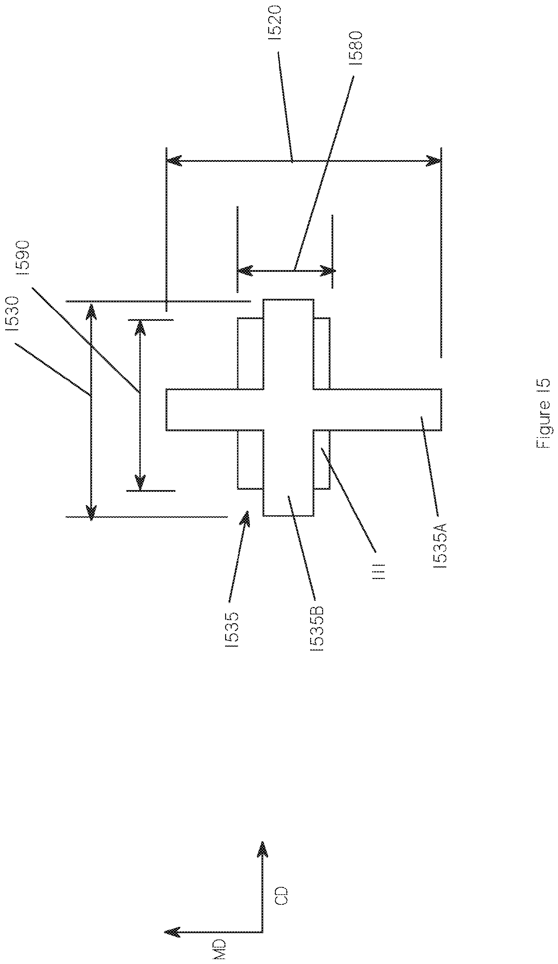

As noted previously, composition sites may be provided to the web in a variety of configurations which are described herein. Some additional configuration are provided with regard to Figure 15. As shown, composition sites 1535 may comprise first portion 1535A and a second portion 1535B. The first portion 1535A may have a first portion length 1520 generally parallel to the MD, and the second portion 1535B may have a second portion width 1530 generally parallel to the CD. Similarly, the discontinuity 111 may comprise a discontinuity length 1580 generally parallel to the MD, and a discontinuity width 1590 generally parallel to the CD. The composition sites 1535 described with regard to Figure 15A, may be similarly configured for the discontinuities described herein.

In some forms of the present invention, the second portion width 1530 may be greater than the first portion length 1520. The second portion width 1530 may be greater than the first portion length 1520 in any suitable ratio. Some suitable ratios include about 1.1 to 1.0, about 1.2 to 1.0, about 1.3 to 1.0, about 1.4 to 1.0, about 1.5 to 1.0, about 1.6 to 1.0, about 1.7 to 1.0, about 1.8 to 1.0, about 1.9 to 1.0 about 2.0 to 1.0, about 2.5 to 1.0, or about 2.75 to 1.0, specifically including all ratios within the above and any ranges created thereby.

In some forms of the present invention, the first portion length 1520 may be greater than the second portion width 1530. The first portion length 1520 may be greater than the second portion width 1530 in any suitable ratio. Some suitable ratios include about 1.1 to 1.0, about 1.2 to 1.0, about 1.3 to 1.0, about 1.4 to 1.0, about 1.5 to 1.0, about 1.6 to 1.0, about 1.7 to 1.0, about 1.8 to 1.0, about 1.9 to 1.0 about 2.0 to 1.0, about 2.5 to 1.0, about 2.75 to 1.0, about 3.0 to 1.0, about 3.25 to 1.0, about 3.50 to 1, about 3.75 to 1, about 4.0 to 1.0, about 4.25 to 1, about 4.5 to 1, about 4.75 to 1, or about 5.0 to 1.0 specifically including all ratios within the above and any ranges created thereby.

In some forms, the first portion length 1520 may be less than the discontinuity length 1580. For example, in some forms, the first portion length 1520 may be less than about 90 percent of the discontinuity length 1580, less than about 80 percent, less than about 75 percent, less than about 70 percent, less than about 60 percent, less than about 50 percent, less than about 40 percent, less than about 30 percent, less than about 20 percent, less than about 10 percent, or less than about 5 percent, specifically including all values within the above and any ranges created thereby.

In some forms, the second portion width 1530 may be greater than the discontinuity 1590. For example, in some forms, the second portion width 1530 may be greater than the discontinuity width 1590 by at least 10 percent, at least 20 percent, at least 30 percent, at least 40 percent, at least 50 percent, at least 60 percent, at least 70 percent, at least 80 percent, at least 90 percent at least 100 percent, at least 110 percent, at least 120 percent, at least 130 percent, at least 140 percent, at least 150 percent, at least 160 percent, at least 170 percent, at least 180 percent, at least 190 percent, or at least 200 percent, specifically including all values within the above ranges and any ranges created thereby.

In some forms, the composition site 1535 may cover greater than about 10 percent of the area of the discontinuity 111 , greater than about 20 percent, greater than about 30 percent, greater than about 40 percent, greater than about 50 percent, greater than about 60 percent, greater than about 70 percent, greater than about 80 percent, greater than about 90 percent, greater than about 100 percent, greater than about 110 percent, greater than about 110 percent, greater than about 120 percent, greater than about 130 percent, greater than about 140 percent, greater than about 150 percent, greater than about 160 percent, greater than about 170 percent, greater than about 180 percent, greater than about 190 percent, or greater than about 200 percent, specifically including all values within these ranges and any ranges created thereby.

Additional configurations for composition sites are contemplated. As noted previously, compositions in accordance with the present disclosure may be deposited on the webs described herein via printing. In such forms, the composition sites may comprise a plurality of dots or droplets of composition. With regard to Figure 16, the composition site 1635 is shown comprising a plurality of composition droplets on a discontinuity.

As shown, the composition site 1635 may comprise a first portion 335A and a second portion 335B each of which comprises a plurality of discrete dots. For ease of illustration, the discrete dots have been enlarged along with the discontinuity 111. The first portion 1635A may comprise a first plurality of discrete dots which are spaced apart at a particular dots per inch, "DPI" spacing. The second portion 335B may comprise a second plurality of discrete dots which are spaced apart at a different DPI. In some forms, the DPI of the first plurality of discrete dots may be greater than the DPI of the second plurality of discrete dots.

Such configurations may form a gradient. For example, where the discontinuity is a tuft, the composition is disposed on an inner surface of the tuft, and the composition comprises a surfactant, a higher DPI in the first portion 335A of discrete dots as opposed to the DPI of the second portion 335B of discrete dots would create a hydrophilicity gradient where hydrophilicity increases with decreasing distance from the distal end of the tuft.

Additional, configurations of the composition site 1635 where the DPI of the second portion 335B of discrete dots is greater than the DPI of the first portion 335A of discrete dots are also contemplated. For example, where the composition site 1635 comprises a hydrophobic substance, the DPI of the second portion 335B of discrete dots may be greater than the DPI of the first portion 335A of discrete dots. Such a configuration may create a hydrophobic gradient where hydrophobicity increases with increasing distance from the distal end of the discontinuity. In such forms, the distal end of the discontinuity can be oriented in the positive Z-direction.

Additional configurations are contemplated where the first portion 335A and the second portion 335B comprise different compositions. For example, the first portion 335A of discrete dots may comprise a hydrophilic composition and the second portion 335B of discrete dots may comprise a hydrophobic composition or vice versa.

The compositions applied to the webs described herein may comprise any suitable chemistry known in the art of absorbent articles. Some examples, include hydrophilic, hydrophobic, lotions, blood modifying agents, etc. Additional suitable compositions are disclosed herein.

Some suitable examples of hydrophilic compositions include a surfactant or combination of surfactants with hydrophilic/lyophilic balance number (HLB) of greater than or equal to about 7, more desirably greater than or equal to about 10, and even more desirably, a HLB of greater than or equal to about 14. Hydrophilic agents that do not generally have a measured HLB may also be used.

Some suitable examples of hydrophilic compositions include non-ionic surfactants including esters, amides, carboxylic acids, alcohols, ethers - polyoxyethylene, polyoxypropylene, sorbitan, ethoxylated fatty alcohols, alyl phenol polyethoxylates, lecithin, glycerol esters and their ethoxylates, and sugar based surfactants (polysorbates, polyglycosides). Other suitable nonionic surfactants include: ethoxylates, including fatty acid ester ethoxylates, fatty acid ether ethoxylates, and ethoxylated sugar derivatives (e.g., ethoxylated fatty acid polyesters, ethoxylated fatty acid sorbitan esters, and the like), and the like, as well as combinations comprising at least one of the foregoing. Other suitable examples include anionic surfactants including sulfonates, sulfates, phosphates, alkali metal salts of fatty acids, fatty alcohol monoesters of sulfuric acid, linear alkyl benzene sulfonates, alkyl diphenyloxide sulfonates, lignin sulfonates, olefin sulfonates, sulfosuccinates, and sulfated ethoxylates of fatty alcohols. Other suitable examples include cationic surfactants including amines (primary, secondary, tertiary), quaternary ammoniums, pyridinium, quaternary ammonium salts- QUATS, alkylated pyridinium salts, alkyl primary, secondary, tertiary amines, and alkanolamides. Other suitable examples include zwiterionic surfactants including amino acids and derivatives, amine oxide, betaines, and alkyl amine oxides. Other suitable examples include polymeric surfactants including polyamines, carboxylic acid polymers and copolymers, EO/PO block copolymers, ethylene oxide polymers and copolymers, and polyvinylpyrrolidone. Other suitable examples include silicone surfactants including dimethyl siloxane polymers with hydrophile. And other suitable examples include perfluorocarboxylic acid salts and fluorosurfactants.

The hydrophilic agents that do not generally have a measured HLB may also be used. Such hydrophilic agents may include, without limitation, diols, such as glycols and polyglycols. Suitable nonionic surfactants include, but are not intended to be limited to, C2-8 diols and polyglycols, and the like. Generally, the diol may be glycols (C2 and C3 diols) and polyglycols. The term "polyglycol" refers to a dihydroxy ether formed by dehydration of two or more glycol molecules. A representative, non-limiting list of useful polyglycols, includes: ethylene glycol, propylene glycol, polyethylene glycols, polypropylene glycols, methoxypolyethylene glycols, polybutylene glycols, block copolymers of butylene oxide and ethylene oxide, and the like, as well as combinations comprising at least one of the foregoing.

Additionally, suitable philic composition include finishing treatments which are typically proprietary blends of synthetic surfactant solutions which are commercially available. Examples include materials from Schill & Seilacher AG under the tradename Silastol (e.g. Silastol PHP 26, Silastol PHP 90, Silastol PST-N, Silastol PHP 207, Silastol PHP 28 & Silastol PHP 8), from Pulcra Chemicals under the tradename Stantex® (e.g. Stantex S 6327,Stantex S 6087-4, & Stantex PP 602), among others.

Some suitable examples of hydrophobic compositions include fluorinated or perfluorinated polymers; silicones; fluorochemicals; zirconium compounds; oils; latexes; waxes; crosslinking resins; and blends thereof; fluorochemical urethanes, ureas, esters, ethers, alcohols, epoxides, allophanates, amides, amines (and salts thereof), acids (and salts thereof), carbodiimides, guanidines, oxazolidinones, isocyanurates, and biurets; nanostructured particles selected from fumed silica, hydrophobic titania, zinc oxide, nanoclay, and mixtures thereof; fats and oils, glycerol derivatives; hydrophobic silicones or suitable combinations thereof.

Examples of suitable silicone polymers are selected from the group consisting of silicone MQ resins, polydimethysiloxanes, crosslinked silicones, silicone liquid elastomers, and combinations thereof. Polydimethylsiloxanes can be selected from the group consisting of vinyl-terminated polydimethsiloxanes, methyl hydrogen dimethylsiloxanes, hydroxyl-terminated polydimethysiloxanes, organo-modified polydimethylsiloxanes, and combinations thereof, among others.

Other hydrophobic materials suitable for the present invention are well defined and documented in the art. For example, US Patent Application Publication No. 2002/0064639 describes hydrophobic compositions selected from the group consisting of silicones, fluorochemicals, zirconium compounds, oils, latexes, waxes, crosslinking resins, and blends thereof. Representative water repellent fluorochemical compounds described in U.S. Pat. No. 7,407,899 include fluorochemical urethanes, ureas, esters, ethers, alcohols, epoxides, allophanates, amides, amines (and salts thereof), acids (and salts thereof), carbodiimides, guanidines, oxazolidinones, isocyanurates, and biurets. U.S. Pat. No. 6,548,732 describes hydrophobic substances from the group consisting of theobroma oil, cacao butter, cocoa butter, petrolatum, mineral jelly, white mineral oil, dimethicone, zinc oxide preparation, chinese white, zinc white, beeswax, lanolin, jojoba oil and combinations thereof. Additionally, U.S. application Ser. No. 13/193,065, filed Jul. 28, 2011 discusses substrates that exhibit superhydrophobic properties when treated with a composition comprising a hydrophobic component selected from fluorinated polymers, perfluorinated polymers, and mixtures thereof; nano-structured particles selected from fumed silica, hydrophobic titania, zinc oxide, nanoclay, and mixtures thereof; and water for an overall water-based, non-organic solvent. Examples of such compositions and surfaces in U.S. application Ser. No. 13/193,065, filed Jul. 28, 2011 exemplify the superhydrophobic treated surfaces that may be used as the nonwoven topsheet of the present invention.

Additionally waxes and other hydrophobic materials can be used, including petroleum-based emollients; fatty acid esters; polyol polyesters; fatty alcohol ethers; sterols and sterol esters, and their derivatives; triglycerides; glyceryl esters; ceramides; and mixtures thereof. The fatty acids may originate from vegetable, animal, and/or synthetic sources. Some fatty acids may range from a C8 fatty acid to a C30 fatty acid, or from a C12 fatty acid to a C22 fatty acid. In another embodiment, a substantially saturated fatty acid may be used, particularly when saturation arises as a result of hydrogenation of fatty acid precursor. Examples of fatty acid derivatives include fatty alcohols, fatty acid esters, and fatty acid amides.

Suitable fatty alcohols (R-OH) include those derived from C12-C28 fatty acids.
R-OH R=C12-C28 alkyl chain

Suitable fatty acid esters include those fatty acid esters derived from a mixture of C12-C28 fatty acids and short chain (C1-C8, preferably C1-C3) monohydric alcohols preferably from a mixture of C12-C22 saturated fatty acids and short chain (C1-C8, preferably C1-C3) monohydric alcohols. The hydrophobic melt additive may comprise a mixture of mono, di, and/or tri-fatty acid esters. An example includes fatty acid ester with glycerol as the backbone and is shown in formula (1).

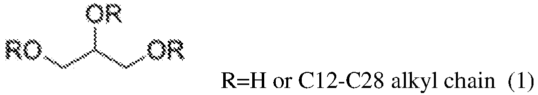

The glycerol derived fatty acid ester has at least one alkyl chain, at least two, or three chains to a glycerol, to form a mono, di, or triglyceride. Suitable examples of triglycerides include glycerol thibehenate (C22), glycerol tristearate (C18), glycerol tripalmitate (C16), and glycerol trimyristate (C14), and mixtures thereof. In the case of triglycerides and diglycerides, the alkyl chains could be the same length, or different length. Example includes a triglyceride with one alkyl C18 chain and two C16 alkyl chain, or two C18 alkyl chains and one C16 chain. Preferred triglycerides include alkyl chains derived from C14-C22 fatty acids.

Suitable fatty acid amides include those derives from a mixture of C12-C28 fatty acids (saturated or unsaturated) and primary or secondary amines. A suitable example of a primary fatty acid amide includes those derived from a fatty acid and ammonia and is shown in formula (2).

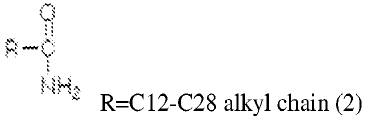

Suitable examples include erucamide, oleamide and behanamide. Other suitable hydrophobic melt additives include hydrophobic silicones, ethoxylated fatty alcohols.

Any suitable lotion may be utilized as a composition of the present invention. Some suitable lotions are described in U.S. Patent Application Publication Nos. 2003/0206943 and 2007/0219515. Lotions suitable for use as compositions in the present invention may comprise from about 60-99.9 percent of a carrier. Suitable carrier compounds include petroleum-based hydrocarbons having from about 8 to about 32 carbon atoms, fatty alcohols having from about 12 to about 18 carbon atoms, polysiloxane compounds, fatty acid esters, alkyl ethoxylates, lower alcohols having from about 2 to about 6 carbon atoms, low molecular weight glycols and polyols, fatty alcohol ethers having from about 12 to about 22 carbon atoms in their fatty chain, lanolin and its derivatives, ethylene glycol derivatives of C₁₂-C₂₂. fatty acids, glyceride and its derivatives including acetoglycerides and ethoxylated glycerides of C₁₂₋C₁₈ fatty acids, and mixtures thereof. Other suitable carriers include oils or fats, such as natural oils or fats, or natural oil or fat derivatives, in particular of plant or animal origin. Suitable carriers further encompass waxes. As used herein, the term 'wax' refers to oil soluble materials that have a waxy constituency and have a melting point or range of above ambient temperature, in particular above 25° C. Waxes are materials that have a solid to semi-solid (creamy) consistency, crystalline or not, being of relative low viscosity a little above their liquefying point. Suitable waxes which can be incorporated into the lotion composition include animal, vegetable, mineral or silicone based waxes which may be natural or synthetic, and including mixtures thereof.

Additionally, lotions suitable for use with the present invention may comprise optional ingredients such as skin treatment agents including hexamidine, zinc oxide, and niacinamide, glycerine, chamomile, panthenol, fats and oils, and/or skin conditioning agents, perfumes, deodorants, opacifiers, astringents, preservatives, emulsifying agents, film formers, stabilizers, proteins, lecithin, urea, colloidal oatmeal, pH control agents. Additional optional ingredients include particles, wetting agents, and/or viscosity or thickening agents.

Additional compositions are contemplated. For example, compositions utilized with the present invention may comprise health actives. Some examples include prebiotics which include mucopolysaccharides, oligosaccharides such as galactooligosaccharides ("GOS"), polysaccharides, amino acids, vitamins, nutrient precursors, harvested metabolic products of biological organisms, lipids, and proteins. Other suitable prebiotics are disclosed in PCT Patent Application Publication No. WO 2013122932 A2. The health actives may be provided to the precursor web or the secondary web independently or in a carrier, e.g. a lotion as described herein.

Other suitable health actives comprise organic acids including acetic acid, propionic acid, lactic acid, ascorbic acid, phenylalanine, citric acid, butyric acid, valeric acid, capronic acid, succinic acid and/or a salt thereof, soluble acrylic acid polymers known to the art as Carbopols ®, alone or in combination with organic acids known to the art such as alphahydroxy acids, more preferably benzoic acid, alginic acid, sorbic acid, stearic acid, oleic acid, edetic acid, gluconodeltalactone, acetic acid, fumaric acid, lactic acid, citric acid, propionic acid, malic acid, succinic acid, gluconic acid, ascorbic acid and tartaric acid and the like.

Other suitable health actives include calcium salts, calcium lactate and/or calcium citrate malate, bacterial metabolites and extracellular products. In some forms, compositions useful with the present invention may comprise skin care actives including allantoin, aluminum hydroxide gel, calamine, cocoa butter, colloidal oatmeal, dimethicone, cod liver oil (in combination), glycerine, hard, fat, kaolin, petrolatum, lanolin, mineral oil, shark liver oil, white petrolatum, sodium bicarbonate, topical starch, zinc acetate, zinc carbonate, zinc oxide, and the like. Additional skin care actives are disclosed in PCT Patent Application Publication No. WO 2013/1222932.

Other suitable health actives include ingredients useful for regulating and/or improving a condition of mammalian skin. Some non-limiting examples of such ingredients include vitamins; peptides and peptide derivatives; sugar amines, phytosterols, salicylic acid compounds, hexamidines, dialkanoyl hydroxyproline compounds, flavonoids, retinoid compounds, botanicals, N-acyl amino acid compounds, their derivatives, and combinations thereof. Other examples include a sugar amine, which is also known as an amino sugar. Exemplary sugar amines suitable for use herein are described in PCT Publication No. WO 02/076423 and U.S. Pat. No. 6,159,485.

Other examples of suitable compositions include a vitamin B3 compound (e.g., niacinamide). Vitamin B3compounds may regulate skin conditions as described in U.S. Pat. No. 5,939,082. Some exemplary derivatives of the foregoing vitamin B3 compounds include nicotinic acid esters, including non-vasodilating esters of nicotinic acid (e.g., tocopheryl nicotinate, myristyl nicotinate). Other examples include a salicylic acid compound, its esters, its salts, or combinations thereof. Still other examples include hexamidine compounds, its salts and derivatives. Other suitable examples include a flavonoid compound. Flavonoids are broadly disclosed in U.S. Pat. Nos. 5,686,082 and 5,686,367.

Additional examples include one or more N-acyl amino acid compounds. The amino acid can be one of any of the amino acids known in the art. A list of possible side chains of amino acids known in the art are described in Stryer, Biochemistry, 1981, published by W.H. Freeman and Company.

Additional examples include a retinoid. "Retinoid" as used herein means natural and synthetic analogs of Vitamin A, or retinol-like compounds which possess the biological activity of Vitamin A in the skin, as well as the geometric isomers and stereoisomers of these compounds.

Other suitable examples may comprise a peptide, including but not limited to, di-, tri-, tetra-, penta-, and hexa-peptides and derivatives thereof. Peptides may contain ten or fewer amino acids and their derivatives, isomers, and complexes with other species such as metal ions (e.g., copper, zinc, manganese, magnesium, and the like). Peptide refers to both naturally occurring and synthesized peptides. Also useful herein are naturally occurring and commercially available compositions that contain peptides.

Compositions of the present invention may also include one or more water-soluble vitamins. Examples of water-soluble vitamins including, but are not limited to, water-soluble versions of vitamin B, vitamin B derivatives, vitamin C, vitamin C derivatives, vitamin K, vitamin K derivatives, vitamin D, vitamin D derivatives, vitamin E, vitamin E derivatives, provitamins thereof, such as panthenol and mixtures thereof.

Other suitable ingredients include a conditioning agent such as a humectant, a moisturizer, or a skin conditioner. Some non-limiting examples of conditioning agents include, but are not limited to, guanidine; urea; glycolic acid and glycolate salts (e.g. ammonium and quaternary alkyl ammonium); salicylic acid; lactic acid and lactate salts (e.g., ammonium and quaternary alkyl ammonium); aloe vera in any of its variety of forms (e.g., aloe vera gel); polyhydroxy alcohols such as sorbitol, mannitol, xylitol, erythritol, glycerol, hexanetriol, butanetriol, propylene glycol, butylene glycol, hexylene glycol and the like; polyethylene glycols; sugars (e.g., melibiose) and starches; sugar and starch derivatives (e.g., alkoxylated glucose, fucose); hyaluronic acid; lactamide monoethanolamine; acetamide monoethanolamine; panthenol; allantoin; and mixtures thereof. Also useful herein are the propoxylated glycerols described in U.S. Pat. No. 4,976,953. Also useful are various C1-C30 monoesters and polyesters of sugars and related materials. These esters are derived from a sugar or polyol moiety and one or more carboxylic acid moieties.

The blood modifying agent of this disclosure can have an IOB of about 0.00-0.60, a melting point of no higher than about 45 deg. C, a water solubility of about 0.00-0.05 g at 25 deg. C, and a weight-average molecular weight of less than about 1,000. The IOB (Inorganic Organic Balance) is an indicator of the hydrophilic-lipophilic balance, and as used herein, it is the value calculated by the following formula by Oda et al. inorganic value/organic value. The inorganic value and the organic value are based on the organic paradigm described in "Organic compound predictions and organic paradigms" by Fujita A., Kagaku no Ryoiki (Journal of Japanese Chemistry), Vol. 11, No. 10 (1957) p. 719-725.

Preferably, the blood modifying agents is selected from the group consisting of following items (i)-(iii), and any combination thereof: (i) a hydrocarbon; (ii) a compound having (ii-1) a hydrocarbon moiety, and (ii-2) one or more, same or different groups selected from the group consisting of carbonyl group ( -- CO -- ) and oxy group ( -- O -- ) inserted between a C -- C single bond of the hydrocarbon moiety; and (iii) a compound having (iii-1) a hydrocarbon moiety, (iii-2) one or more, same or different groups selected from the group consisting of carbonyl group (CO -- ) and oxy group ( -- O -- ) inserted between a C -- C single bond of the hydrocarbon moiety, and (iii-3) one or more, same or different groups selected from the group consisting of carboxyl group ( -- COOH) and hydroxyl group ( -- OH) substituting a hydrogen of the hydrocarbon moiety. As used herein, "hydrocarbon" refers to a compound composed of carbon and hydrogen, and it may be a chain hydrocarbon, such as, a paraffinic hydrocarbon (containing no double bond or triple bond, also referred to as alkane), an olefin-based hydrocarbon (containing one double bond, also referred to as alkene), an acetylene-based hydrocarbon (containing one triple bond, also referred to as alkyne), or a hydrocarbon comprising two or more bonds selected from the group consisting of double bonds and triple bonds, and cyclic hydrocarbon, such as, aromatic hydrocarbons and alicyclic hydrocarbons.

Examples of suitable blood modifying agents include esters of chain hydrocarbon polyols. The polyol may have 2-5 alcohol groups on a 2-5 carbon backbone, and between 1 and 5 of the alcohols may be derivatives with a fatty acid having between 4-22 carbon atoms and 0 to 4 double bonds. Suitable examples include triesters of glycerin and fatty acids, represented by formula (3): 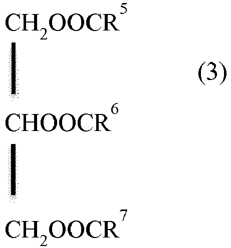 diesters of glycerin and fatty acids, represented by the following formula (4): 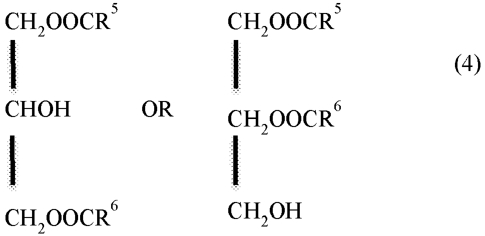 and monoesters of glycerin and fatty acids, represented by the following formula (5): 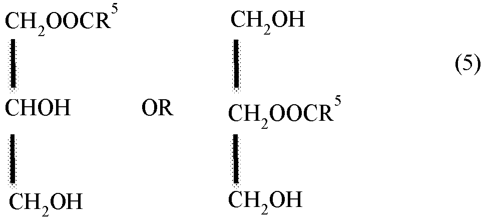 wherein R5-R7 each represent a chain hydrocarbon.

The fatty acid composing the ester of glycerin and a fatty acid (R5COOH, R6COOH and R7COOH) is not particularly restricted so long as the ester of glycerin and a fatty acid satisfies the conditions for the IOB, melting point and water solubility. The esters of glycerin and a fatty acid is preferably a diester or triester, and more preferably a triester. A triester of glycerin and a fatty acid is also known as a triglyceride, and examples include triesters of glycerin and octanoic acid (C8), triesters of glycerin and decanoic acid (C10), triesters of glycerin and dodecanoic acid (C12), triesters of glycerin and 2 or more different fatty acids, and mixtures of the foregoing. Examples of triesters of glycerin and 2 or more fatty acids include triesters of glycerin with octanoic acid (C8) and decanoic acid (C10), triesters of glycerin with octanoic acid (C8), decanoic acid (CH) and dodecanoic acid (C12), and triesters of glycerin with octanoic acid (C8), decanoic acid (C10), dodecanoic acid (C12), tetradecanoic acid (C14), hexadecanoic acid (C16) and octadecanoic acid (C18).

Blood modifying agents are described further in U.S. Patent Application Publication No. 2014/0358102 and U.S. Patent No. 9,248,060. In some forms of the present disclosure, it may be beneficial, particularly from a cost standpoint, if the blood modifying agent were applied in a plurality of discrete areas on a web as opposed to a uniform coating of the web.

The absorbent articles of the present disclosure -- particularly the topsheets of the absorbent articles - may comprise a myriad of compositions to provide a wide range of benefits to a user of the absorbent articles. However, depending on the manner in which the compositions are provided to the web, it is important to consider the rheology of the compositions being applied. For example, viscosity of the composition can be an important factor as viscosities which are too low can migrate out of the applied area, e.g. first composition sites. In contrast, a composition with too high of a viscosity can be difficult to apply via digital printer. And, other forms of application of the composition may prove to be much slower than that of the digital printer.

The composition of the present invention may be formulated to optimize its deposition by non-contact printing, e.g. ink jet printing. For example, the components of the desired composition can be dissolved or dispersed in a suitable solvent, such as water or another organic solvent. Some suitable organic solvents include ketones such as acetone, diethyl ketone, cyclophexanone and the like. Additional suitable solvents include alcohols such as methanol, ethanol, n-propanol, isopropanol, n-butanol, 1-methoxy-2-propanol, and the like. Additional suitable solvents include esters such as ethyl acetate, propyl acetate, butyl acetate and the like. Additional examples include ethers, lactones and amides. If desired, a mixture of solvents may be used. Additionally surfactants, rheology modifiers, and colorants such as dyes or pigments may be added to the formulation.

Additional forms are contemplated where the compositions to be deposited can be heated such that the viscosity of the composition is provided in the correct range for deposition via ink jet. For example, heated print heads are available from Fujifilm Dimatix under the trade name Galaxy PH256/80HM.

Ink jet printing generally relies on the generation of sequences of droplets. Behavior of the composition during droplet ejection is dependent on material properties such as density, viscosity and surface tension. The behavior of a composition when ink jet printed can be predicted via two dimensionless numbers, i.e. Ohnesorge number and Weber number. The equation for determining the Oh number is provided below. 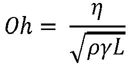 where η is viscosity, ρ is density, y is surface tension of the composition, and L is the characteristic diameter (print head nozzle diameter for ink jet printing in meters).

Stable drop formation can be characterized by the reciprocal of the Ohnesorge number, namely Z = 1/Oh. Stable drop formation can be expected from compositions when 14 ≥ Z ≥ 1. The viscosity of the desired composition should be measured at target operating temperature with shear rates between 200 and 20 s-1. The surface tension should be recorded in N/m. The density should be calculated in kg/m3, and the viscosity should be recorded in Pa·s.

Additionally, a composition of the present invention may comprise a Weber number of between about 4 and 1000. The Weber number may be calculated as follows: 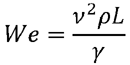 where ρ is the density of the composition in kg/m3; v is the velocity of the composition in m/s; L is the characteristic diameter (print head nozzle diameter for ink jet printing; and y is the surface tension in N/m.

The compositions of the present invention may comprise a viscosity of between about 5 and 25 centipoise. The compositions may comprise a surface tension of between about 25 and 40 dyne. In some forms of the present invention, the compositions may comprise a density of from about 0.6 grams / cubic cm to about 2.0 grams / cubic cm, specifically including all values within this range and any ranges created thereby.

### Equipment:

The camera 131 can be fixed with respect to a manufacturing line such that the centerline of the camera 131 is co-linear with the machine centerline 130. In some forms, the centerline of the camera 131 is not co-linear with the machine centerline 130 but utilizes the machine centerline 130 and/or another fixed reference.

Any suitable camera may be utilized. For example, a camera having a bit depth of at least 8 may be utilized. In another example, a camera having a bit depth of at least 12 or at least 16 may be utilized. Cameras with higher bit depth can provide the computational device with much more numerical resolution allowing for better filtering of images by the computational device.

Any suitable computational device may be utilized with the present invention. Some suitable examples can include central processing units (CPU), graphical processing units (GPU), and/or field programmable gate arrays (FPGA). The processing power / speed of the computational device may vary depending on the speed of the manufacturing line of which images are being provided to the computational device. For example, faster line speeds may require additional processing power to ensure that the computational device can keep up with the images being provided by the camera. In some forms of the present invention, manufacturing line speeds can be greater than about 1 m/s, greater than about 3 m/s, greater than about 4 m/s, greater than about 5 m/s, greater than about 6 m/s, greater than about 7 m/s, greater than about 8 m/s, greater than about 9 m/s, greater than about 10 m/s, greater than about 11 m/s, greater than about 12 m/s, greater than about 13 m/s or greater than about 14 m/s specifically including all values within the above values and any ranges created thereby.

The computational device can comprise any suitable vision analysis software. Some suitable examples include National Instruments® Vision Development Module, MathWorks® Image Processing toolkit, OpenCV - open source computer vision library written in C++, or ImageJ. The vision analysis software can allow a user to extract a Fourier plane from the image provided by the camera and extract the phase plane from the image provided by the camera. Depending on the intermediate features and/or discontinuities being analyzed, settings may need to be adjusted. For example, apertures may be difficult to discern in low basis weight nonwovens without adjustment to the filtering to reduce the noise of the image signal. However, less filtering may be required for the same size apertures in a higher basis weight nonwoven. Samples of the images to be analyzed can be used in test runs to hone the filter settings and produce a signal which can provide accurate results.

Similarly, samples may be utilized to determine the best highlighting method for the discontinuities. For example, backlighting may be used to highlight apertures. However, backlighting may not provide good results for highlighting the discontinuities described herein. In general, the discontinuities herein may be highlighted via laser topography. Where compositions are desired to be highlighted, thermal imaging may be effective at highlighting compositions on webs described herein.

As noted previously, the vision analysis software can allow analysis of an image via the Fourier and phase plane of the image. Additionally, the vision analysis software can allow for comparisons between predetermined patterns and images from the camera - pattern recognition. Where the periodicity of the discontinuities is too disparate, Fourier analysis may not be appropriate. In such instances, pattern recognition may provide more accurate results / more accurate instructions to the printer. A pattern or a plurality of patterns of discontinuities would need to be provided to the computational device and/or printer such that the comparison could be made between the transmitted image and the stored pattern(s).

For pattern recognition, a plurality of patterns may be stored in the computational device and/or printer to address potential phase shift of the pattern with respect to its web. The plurality of patterns may account for phase shifts of the intermediate features and/or discontinuities in the web.

Configurations are contemplated where the camera provides an image to the computational device which then creates a print file from the image. The print file can then be provided to the printer without the need for analysis. For example, the print file can account for any phase shift in the MD or CD. In this form, the need for predetermined patterns may be obviated.

Any suitable printer may be utilized with the present invention. As noted previously, the composition sites may comprise a plurality of discrete dots or droplets. The volume of the composition droplets can depend on the particular printing technology. By way of example, printing units that are VIDEOJET™ continuous ink jet printers can have composition drop volumes of about 240 pL and are delivered at relatively high drop velocities (e.g., about 13 m/s). Other printing technology (e.g. piezo drop on demand) can deliver composition drops having relatively small volumes, such as composition drops having a volume ranging from about 1 pL to about 80 pL, that are delivered at lower drop velocities (i.e., about ½ m/s) than continuous ink jet printing. Those skilled in the art know there are different ink jet technologies (e.g., continuous, piezo, thermal, valve) and different drop size ranges and different jet velocities. In general, smaller drop size infers that the CD dpi (resolution) is higher. The range 1-24 pL would equate to a CD resolution of 300-600 dpi. The VIDEOJET CD resolution is 128 dpi. So, more drops in CD can mean better opportunity to hit a fiber, which can result in better image quality and less composition blow-though. The slower the drop speed, the less composition blow-through.

An exemplary continuous ink jet printer is available from Videojet™ sold under the trade name of Videojet BX™. For the continuous ink jet printer, the composition droplets are dispensed from all of the jets of the print heads continuously, but only certain composition droplets are allowed to reach the precursor web, intermediate web, or secondary web, at the composition sites. The other composition droplets can be prevented from reaching the precursor web, intermediate web, or secondary web by deflecting the composition droplets into a recycling flow for a continuous re-use. The operation of the individual ink jets of each print head can be controlled by a controller included in the Videojet BX™ system.

Exemplary drop on demand printers for use in the present invention may comprise multiple print heads allowing for the deposition of a plurality of compositions. In general, the printer of the present invention may comprise a controller, one or more print heads, and a composition management system. A suitable example of a printer includes the 1024 PH development kit available from FujiFilm Dimatix™ located in New Hampshire. A suitable example of the print heads which may be utilized, includes SG-1024 MA available from FujiFilm Dimatix™. Forms of the present invention are contemplated where the controller 120 (See Figures 1A, 2, 4A, and 4D) is utilized as the controller for the printer described above. Additional forms are contemplated where the printer described above comprises a separate controller in addition to the controller 120. Still in other forms of the present invention, where the need for a vision system is optional based upon the above disclosure, the controller for the printer may operate without the controller 120.

### Disposable Absorbent Articles

The webs of the present invention may be processed to a further extent to create disposable absorbent articles. Some suitable examples include diapers, diaper pants, feminine pads, adult incontinence pads, etc. The webs of the present invention may form any suitable portion of a disposable absorbent article. For example, the webs of the present invention may form a portion of a topsheet, a backsheet, or an absorbent core which is disposed between the toposheet and the backsheet. In some forms, the webs of the present invention may be utilized to form barrier cuffs for a disposable absorbent article. In other forms, the webs of the present invention may form a portion of at least one or more of the topsheet, backsheet, secondary topsheet, acquisition layer, distribution layer, absorbent core dusting layer, backsheet, barrier cuff, wing of a sanitary pad, ear on a diaper, or the like.

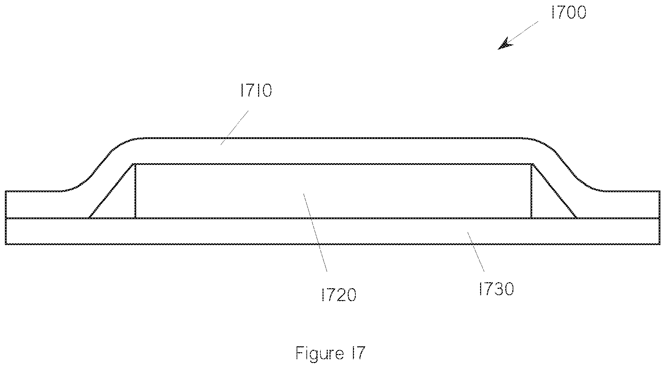

An exemplary disposable absorbent article is shown with regard to Figure 17. With regard to Figure 17 a cross sectional view of disposable absorbent article 1700 is shown. The disposable absorbent article 1700 may comprise a topsheet 1710, a backsheet 1730, and an absorbent core 1720 disposed therebetween. Optional features include, barrier cuffs, gasketing cuffs, wings, or the like.

The topsheet, backsheet, and/or absorbent core may comprise any suitable materials. Exemplary materials are disclosed in U.S. Patent Application Publication Nos. 2016/0167334A1; 2016/0129661A1; 2016/0136014A1; and U.S. Application Serial No. 62/076043.

### Test Methods

Various values are reported herein for the purposes of characterizing the invention. The methods for their determination are detailed below.

Distinguishing a printed dot pattern from a continuous composition layer is done by viewing a substrate under a magnified condition using a light microscope. Evidence of a printed dot pattern would include the presence of: clusters of discrete circular colored regions, regular pattern of interior non-printed regions, clusters of discrete circular regions which are a different color than the primary color of the substrate, and scalloped or rounded edges present at the boundaries between printed and non-printed regions. Areas of interest, defined below, may be viewed as described above for the appearance of printed dot patterns. As noted herein, some particular areas of interest include distal ends (both outer surface and inner surface) of discontinuities, sidewalls of discontinuities (both outer surface and inner surface), and land areas between adjacent discontinuities or openings.

### Contact Angle Method

Contact angles on substrates are determined using ASTM D7490-13 modified with the specifics as described herein, using a goniometer and appropriate image analysis software (a suitable instrument is the FTA200, First Ten Angstroms, Portsmouth, VA, or equivalent) fitted with a 1 mL capacity, gas tight syringe with a No. 27 blunt tipped stainless steel needle. One test fluid is used: Type II reagent water (distilled) in accordance with ASTM Specification D1193-99. All testing is to be performed at about 23 °C ± 2 C° and a relative humidity of about 50% ± 2%.

A 50 mm by 50 mm substrate to be tested is removed from the article taking care to not touch the region of interest or otherwise contaminate the surface during harvesting or subsequent analysis. Condition the samples at about 23 °C ± 2 C° and a relative humidity of about 50% ± 2% for 2 hours prior to testing.

Set up the goniometer on a vibration-isolation table and level the stage according to the manufacturer's instructions. The video capture device must have an acquisition speed capable of capturing at least 10-20 images from the time the drop hits the surface of the specimen to the time it cannot be resolved from the specimen's surface. A capture rate of 900 images/sec is typical. Depending on the hydrophobicity / hydrophilicity of the specimen, the drop may or may not rapidly wet the surface of the sample. In the case of slow acquisition, the images should be acquired until 2% of the volume of the drop is absorbed into the specimen. If the acquisition is extremely fast, the first resolved image should be used if the second image shows more than 2% volume loss.

Place the specimen on the goniometer's stage and adjust the hypodermic needle to the distance from the surface recommended by the instrument's manufacturer (typically 3 mm). If necessary adjust the position of the specimen to place the target site under the needle tip. Focus the video device such that a sharp image of the drop on the surface of the specimen can be captured. Start the image acquisition. Deposit a 5 µL ± 0.1 µL drop onto the specimen. If there is visible distortion of the drop shape due to movement, repeat at a different, but equivalent, target location. Make two angle measurements on the drop (one on each drop edge) from the image at which there is a 2% drop volume loss. If the contact angles on two edges are different by more than 4°, the values should be excluded and the test repeated at an equivalent location on the specimen. Identify ten additional equivalent sites on the specimen and repeat for a total of 11 measurements (22 angles). Calculate the arithmetic mean for this side of the specimen and report to the nearest 0.01°. In like fashion, measure the contact angle on the opposite side of the specimen for 11 drops (22 angles) and report separately to the nearest 0.01°.

For any sites which demonstrate an arithmetic mean which is higher or lower than another arithmetic mean -- by at least 2 times the highest standard deviation the angle measurements comprised by the two arithmetic means - an equivalent site on a specimen from another article shall be measured in accordance to the SEM Method for determining contact angle on fibers. Any such sites shall be termed "area of interest."

Moreover, when an area of interest of the specimen is on a distal end and/or sidewall of a protrusion, the contact angle measurements with regard to the distal end and/or sidewall shall be performed in accordance with the SEM Method for determining contact angle on fibers described herein.

### SEM Method for determining contact angle on fibers

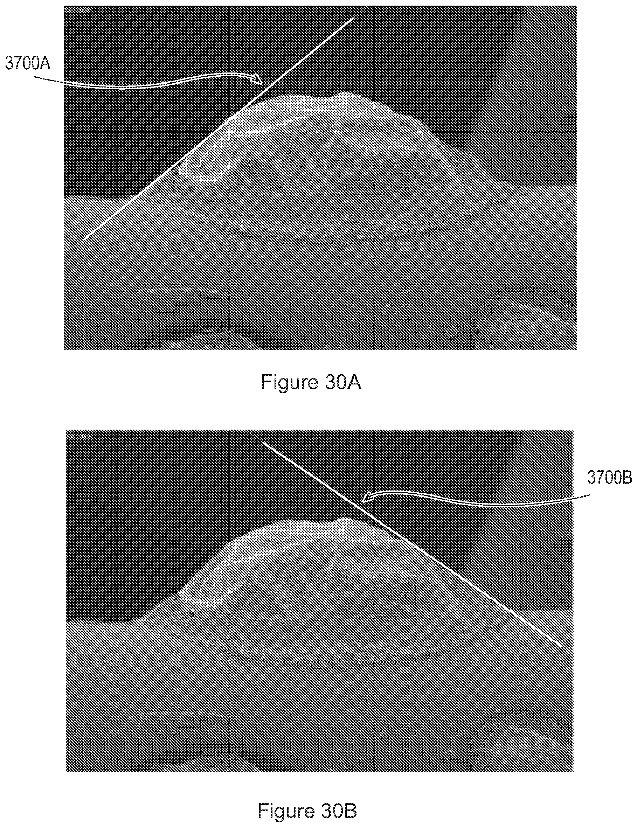

A rectangular specimen measuring 1 cm x 2 cm is cut from the topsheet of a disposable absorbent product taking care not to touch the surface of the specimen or to disturb the structure of the material. The specimen shall include the area of interest determined in the Contact Angle Method heretofore described. If multiple areas of interest are identified then additional specimens shall be obtained in accordance with this method to accommodate all areas of interest identified. The specimen has a length of (2 cm) aligned with a longitudinal centerline of the article. The specimen is handled gently by the edges using forceps and is mounted flat with the skin-facing side up on an SEM specimen holder using double-sided tape. The specimen is sprayed with a fine mist of water droplets generated using a small hobby air-brush apparatus. The water used to generate the droplets is distilled deionized water with a resistivity of at least 18 MΩ-cm. The airbrush is adjusted so that the droplets each have a volume of about 2 pL. Approximately 0.5 mg of water droplets are evenly and gently deposited onto the specimen. Immediately after applying the water droplets, the mounted specimen is frozen by plunging it into liquid nitrogen. After freezing, the sample is transferred to a Cryo-SEM prep chamber at -150°C, coated with Au/Pd, and transferred into Cryo-SEM chamber at -150°C. A Hitachi S-4700 Cry-SEM or equivalent instrument is used to obtain high-resolution images of the droplets on the fibers. Droplets are randomly selected, though a droplet is suitable to be imaged only if it is oriented in the microscope such that the projection of the droplet extending from the fiber surface is approximately maximized. This is further discussed with regard to Figures 30-33. The contact angle between the droplet and the fiber is determined directly from the images taken as is shown via lines 3700A, 3700B, 3800A, 3800B, 3900A, 3900B, 4000A, and 4000B. Twenty separate droplets are imaged from which forty contact angle measurements are performed (one on each side of each imaged droplet), and the arithmetic average of these forty contact angle measurements is calculated and reported as the contact angle for that specimen.

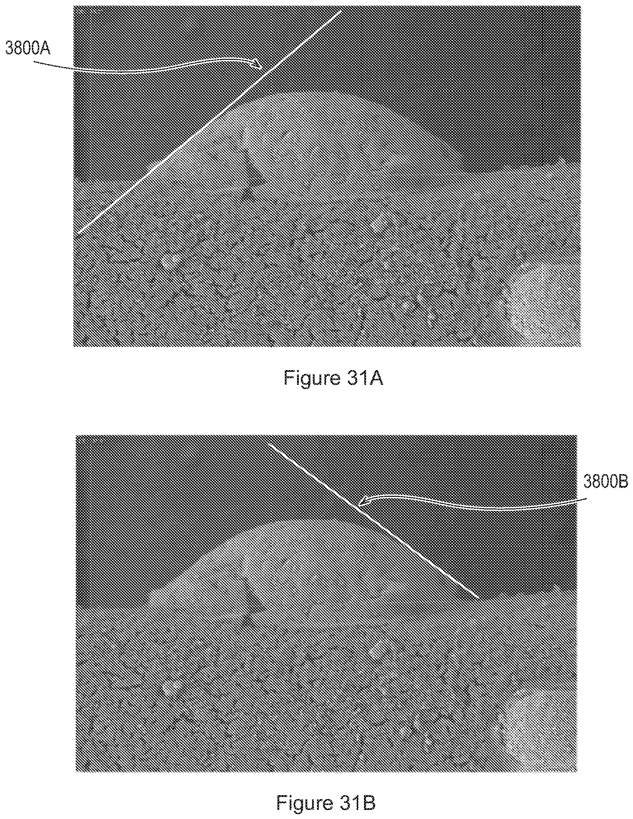

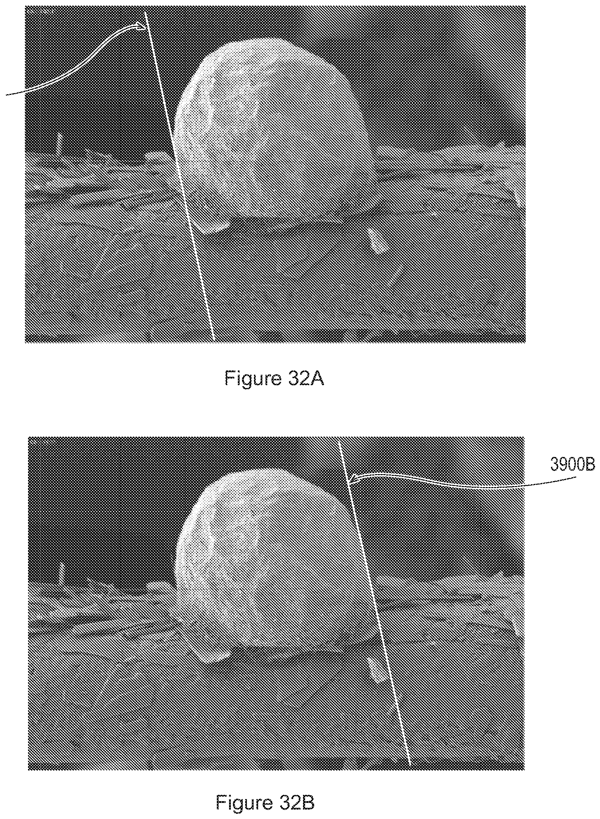

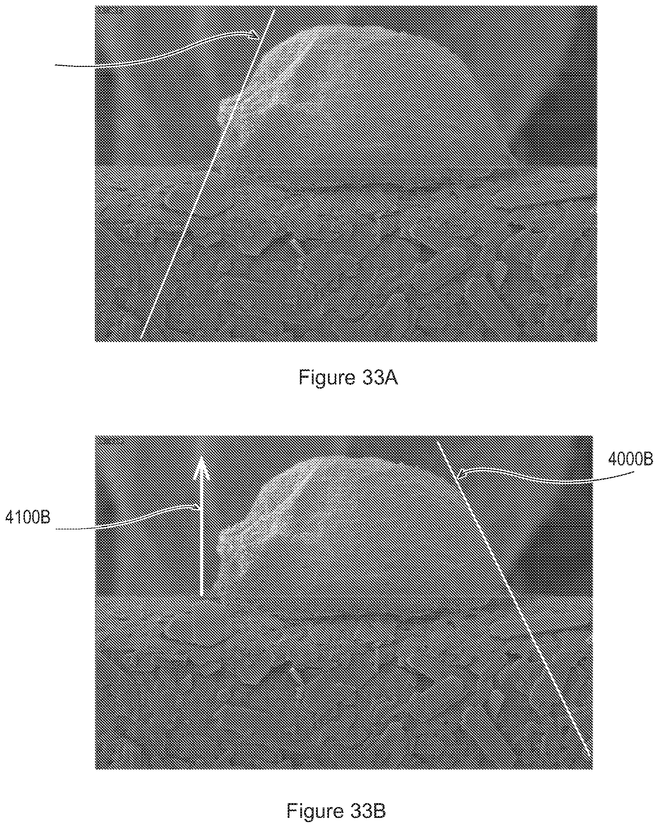

Examples of images are provided with regard to Figures 30-33. Figures 30 and 31 are exemplary images depicting water droplets cryogenically frozen on fibers upon which no composition has been applied. Figures 32 and 33 are exemplary images depicting water droplets cryogenically frozen on fibers upon which composition has been applied. As noted previously, the projection of the droplet should be maximized to ensure that the appropriate contact angle is measured. An exemplary droplet projection 4100B is shown in Figure 33B.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm."

## Claims

1. An absorbent article (421, 1700) comprising a topsheet (1710), a backsheet (455, 1730), and an absorbent core (465, 1720) disposed between the topsheet and the backsheet, the absorbent article comprising:
a web (180) having a first surface (225) and an opposing second surface (237), the web comprising a plurality of discontinuities (111) each having a distal end (354, 411C, 554, 555, 654, 754, 854, 2632, 2742, 3754) and sidewalls (356, 411A, 411B, 556, 557, 656, 756, S, 2636, 3756) joining the distal end to the first surface or the second surface; a plurality of openings (385) corresponding to the discontinuities, wherein openings are disposed opposite distal ends of the discontinuities; a plurality of land areas (340) disposed between adjacent discontinuities and adjacent openings; and wherein the web further comprises a plurality of first composition sites (235A, 235B, 235C, 235D) comprising a plurality of composition dots, wherein at least a portion of the distal ends or at least a portion of the land areas of the web comprise first composition sites;
wherein the distal ends are oriented in a negative Z-direction away from a wearer-facing surface of the absorbent article such that the distal ends are subjacent to the first surface, and
wherein the web forms a portion of the topsheet of the absorbent article.

2. The absorbent article of claim 1, wherein the plurality of first composition sites is more hydrophobic than constituent material of the web and wherein the plurality of first compositions sites is disposed on a portion of the land areas between adjacent openings.

3. The absorbent article of claim 1, wherein the plurality of first composition sites is more hydrophilic than constituent material of the web and wherein the plurality of first compositions sites is disposed on an inner surface of distal ends of the plurality of discontinuities.

4. The absorbent article of claim 2, wherein at least a portion of the distal ends comprise a second composition site disposed on an inner surface of the distal ends.

5. The absorbent article of claim 4, wherein the second composition site is more hydrophilic than the plurality of first composition sites.

6. The absorbent article of any of the preceding claims, wherein the web further comprises a plurality of apertures extending from the first surface through the second surface.

7. The absorbent article of claim 6, wherein the apertures comprise variable sizes and/or are arranged at differing angles.

8. The absorbent article of any of the preceding claims, wherein the discontinuities comprise ridges and grooves, tufts, filled tufts, and/or nested tufts.

## Patentansprüche

1. Absorptionsartikel (421, 1700), umfassend eine Oberschicht (1710), eine Unterschicht (455, 1730) und einen zwischen der Oberschicht und der Unterschicht angeordneten Absorptionskern (465, 1720), wobei der Absorptionsartikel Folgendes umfasst:
eine Bahn (180) mit einer ersten Oberfläche (225) und einer gegenüberliegenden zweiten Oberfläche (237), wobei die Bahn eine Vielzahl von Unstetigkeiten (111) umfasst, die jeweils ein distales Ende (354, 411C, 554, 555, 654, 754, 854, 2632, 2742, 3754) und Seitenwände (356, 411A, 411B, 556, 557, 656, 756, S, 2636, 3756) aufweist, die das distale Ende mit der ersten Oberfläche oder der zweiten Oberfläche verbinden; eine Vielzahl von Öffnungen (385), die den Unstetigkeiten entspricht, wobei Öffnungen entgegengesetzt von distalen Enden der Unstetigkeiten angeordnet sind; eine Vielzahl von Auftreffbereichen (340), die zwischen benachbarten Unstetigkeiten und benachbarten Öffnungen angeordnet ist; und wobei die Bahn ferner eine Vielzahl von ersten Zusammensetzungsstellen (235A, 235B, 235C, 235D) umfasst, umfassend eine Vielzahl von Zusammensetzungspunkten, wobei mindestens ein Abschnitt der distalen Enden oder mindestens ein Abschnitt der Auftreffbereiche der Bahn erste Zusammensetzungsstellen umfasst;
wobei die distalen Enden in einer negativen Z-Richtung weg von einer trägerseitigen Oberfläche des Absorptionsartikels ausgerichtet sind, sodass die distalen Enden unter der ersten Oberfläche liegen, und
wobei die Bahn einen Abschnitt der Oberschicht des Absorptionsartikels bildet.

2. Absorptionsartikel nach Anspruch 1, wobei die Vielzahl von ersten Zusammensetzungsstellen hydrophober ist als Bestandteilmaterial der Bahn und wobei die Vielzahl von ersten Zusammensetzungsstellen auf einem Abschnitt der Auftreffbereiche zwischen benachbarten Öffnungen angeordnet ist.

3. Absorptionsartikel nach Anspruch 1, wobei die Vielzahl von ersten Zusammensetzungsstellen hydrophiler ist als Bestandteilmaterial der Bahn und wobei die Vielzahl von ersten Zusammensetzungsstellen auf einer Innenoberfläche von distalen Enden der Vielzahl von Unstetigkeiten angeordnet ist.

4. Absorptionsartikel nach Anspruch 2, wobei mindestens ein Abschnitt der distalen Enden eine zweite Zusammensetzungsstelle umfasst, die auf einer Innenfläche der distalen Enden angeordnet ist.

5. Absorptionsartikel nach Anspruch 4, wobei die zweite Zusammensetzungsstelle hydrophiler ist als die Vielzahl von ersten Zusammensetzungsstellen.

6. Absorptionsartikel nach einem der vorstehenden Ansprüche, wobei die Bahn ferner eine Vielzahl von Öffnungen umfasst, die sich von der ersten Oberfläche durch die zweite Oberfläche erstreckt.

7. Absorptionsartikel nach Anspruch 6, wobei die Öffnungen variable Größen umfassen und/oder in unterschiedlichen Winkeln angeordnet sind.

8. Absorptionsartikel nach einem der vorstehenden Ansprüche, wobei die Unstetigkeiten Kämme und Rillen, Büschel, gefüllte Büschel und/oder verschachtelte Büschel umfassen.

## Revendications

1. Article absorbant (421, 1700) comprenant une feuille de dessus (1710), une feuille de fond (455, 1730), et une âme absorbante (465, 1720) disposée entre la feuille de dessus et la feuille de fond, l'article absorbant comprenant :
une nappe (180) ayant une première surface (225) et une deuxième surface opposée (237), la nappe comprenant une pluralité de discontinuités (111) ayant chacune une extrémité distale (354, 411C, 554, 555, 654, 754, 854, 2632, 2742, 3754) et des parois latérales (356, 411A, 411B, 556, 557, 656, 756, S, 2636, 3756) joignant l'extrémité distale à la première surface ou à la deuxième surface ; une pluralité d'ouvertures (385) correspondant aux discontinuités, dans lequel des ouvertures sont disposées opposées à l'extrémité distale des discontinuités ; une pluralité de zones plates (340) disposées entre des discontinuités adjacentes et des ouvertures adjacentes ; et dans lequel la nappe comprend en outre une pluralité de sites de première composition (235A, 235B, 235C, 235D) comprenant une pluralité de points de composition, dans lequel au moins une partie des extrémités distales ou au moins une partie des zones plates de la nappe comprennent des sites de première composition ;
dans lequel les extrémités distales sont orientées dans une direction Z négative à l'écart d'une surface faisant face au porteur de l'article absorbant de telle sorte que les extrémités distales sont sous-jacentes à la première surface, et
dans lequel la nappe forme une partie de la feuille de dessus de l'article absorbant.

2. Article absorbant selon la revendication 1, dans lequel la pluralité de sites de première composition est plus hydrophobe qu'un matériau constitutif de la nappe et dans lequel la pluralité de sites de première composition est disposée sur une partie des zones plates entre des ouvertures adjacentes.

3. Article absorbant selon la revendication 1, dans lequel la pluralité de sites de première composition est plus hydrophile qu'un matériau constitutif de la nappe et dans lequel la pluralité de sites de première composition est disposée sur une surface interne d'extrémités distales de la pluralité de discontinuités.

4. Article absorbant selon la revendication 2, dans lequel au moins une partie des extrémités distales comprennent un site de deuxième composition disposé sur une surface interne des extrémités distales.

5. Article absorbant selon la revendication 4, dans lequel le site de deuxième composition est plus hydrophile que la pluralité de sites de première composition.

6. Article absorbant selon l'une quelconque des revendications précédentes, dans lequel la nappe comprend en outre une pluralité d'ouvertures s'étendant à partir de la première surface à travers la deuxième surface.

7. Article absorbant selon la revendication 6, dans lequel les ouvertures comprennent des tailles variables et/ou sont agencées selon des angles différents.

8. Article absorbant selon l'une quelconque des revendications précédentes, dans lequel les discontinuités comprennent des crêtes et des rainures, des touffes, des touffes remplies, et/ou des touffes imbriquées.
